# EUROPEAN PATENT APPLICATION

(11) **EP 4 032 526 A1**
(43) Date of publication of application: **27.07.2022**
(21) Application number: 21198738.3
(22) Date of filing: 07.09.2018
(51) Int. Cl.: A61K 9/00, A61K 35/76, C12N 7/00

(54) **BACTERIOPHAGE FOR MODULATING INFLAMMATORY BOWEL DISEASE**

(30) Priority: 08.09.2017 US 201762555790 P
(62) Divisional of application: 18803755.0
(71) Applicant: Keio University, Tokyo 108-8345 (JP); Biomx Ltd., 7403635 Ness Ziona (IL); Yeda Research and Development Co. Ltd. at the Weizmann Institute of Science, 76100 Rehovot (IL)
(72) Inventor: HONDA, Kenya, Tokyo, 160-8582 (JP); ATARASHI, Koji, Tokyo, 160-8582 (JP); NARUSHIMA, Seiko, Tokyo, 160-8582 (JP); ELINAV, Eran, 7680400 Mazkeret Batia (IL); SOREK, Rotem, 76100 Rehovet (IL); KHABRA, Efrat, 491606 Petach Tikva (IL); BEN DAVID, Hava, 7624811 7624811 (IL); WEINSTOCK, Eyal, 66083 Tel Aviv (IL); POLLOCK, Sarah, 7628920 Rehovot (IL); MUTIUHIN, Yulia, 37105 Pardess Hanna Karkur (IL); ZAK, Naomi, 945611 Jerusalem (IL)
(74) Representative: Gitto, Serena

(57) **Abstract**

Disclosed herein are bacteriophage compositions and therapeutic uses thereof. The disclosure also relates to bacteriophage that are capable of lysing Klebsiella bacterial strains, e.g., strains that are associated with inflammatory bowel disease, and thereby capable of modulating disease.

## Description

### Background

This application claims priority to U.S. Provisional Application No. 62/555,790, filed September 8, 2017, the contents of which are incorporated by reference herein in its entirety.

The instant application contains a Sequence Listing which has been filed electronically in ASCII format and is hereby incorporated by reference in its entirety. Said ASCII copy, created on August 31, 2017, is named 14283_6000-00000_SL.txt and is 32,895,979 bytes in size.

This disclosure relates to bacteriophage compositions and therapeutic uses thereof. In a particular aspect, the disclosure relates to lytic bacteriophages that are capable of lysing *Klebsiella* bacterial strains. In certain embodiments, the lytic bacteriophages are capable of lysing *Klebsiella* bacterial strains that are associated with inflammatory bowel disease (IBD) (e.g., ulcerative colitis, Crohn's disease), thereby modulating the disease.

Gut microbiota dysbiosis is associated with the pathogenesis of IBD (Said et al.). Increased stimulation from the gut microbiota can result in inflammation and alter a patient's immune response. In humans, the oral microbiota comprises over 700 species or phylotypes of bacteria (Said et al.). Ingested oral bacteria do not typically colonize the healthy intestine (Chen et al., 2016; Bik et al., 2010; U.S. Provisional Application No. 62/415,759, TH1 Cell Stimulatory Bacteria Colonizing in Human Oral Cavity; *see also* Example 1). However, bacteria of oral origin may be increased in the gut microbiota of patients with IBD (Gevers et al., 2014). Oral microbiota dysbiosis is observed in patients with IBD and could contribute to disease etiology (Said et al.).

IBDs, such as Crohn's disease (CD) and ulcerative colitis (UC), can cause diarrhea, malabsorption of nutrients, malnutrition, anemia, and weight loss. Severe intestinal inflammation can extend beyond the mucosa of the intestines and cause ulceration, bleeding, toxic megacolon, strictures, and fistulas. In addition, chronic inflammation is associated with colon cancer. Extraintestinal complications include, but are not limited to, arthritis, skin rashes, liver disease, and eye disorders such as episcleritis and uveitis.

According to the Crohn's & Colitis Foundation of America, "there is no single ideal therapy" for IBD. Dietary restriction alone has not been shown to ameliorate IBD. For many patients, surgical intervention is necessary. Even with the currently available IBD treatments, many patients experience IBD flares between periods of remission. Thus, there is significant unmet need for effective, reliable, and long-term treatment and/or prevention of IBD.

The disclosure provides bacteriophage compositions and therapeutic uses thereof. In specific embodiments, the invention provides lytic bacteriophages that are capable of lysing one or more *Klebsiella* species, strains, or substrains, such as *Klebsiella pneumoniae* (e.g., *Klebsiella pneumoniae* 2H7 strain, "KP2" herein) or *Klebsiella aeromobilis* (e.g., *Klebsiella aeromobilis*/*Enterobacter aerogenes* 11E12, "KP3" herein), that are associated with IBD. The disclosure provides methods for modulating IBD. The disclosure also provides methods for selecting patients to be treated with the methods provided herein.

### Brief Description of the Figures

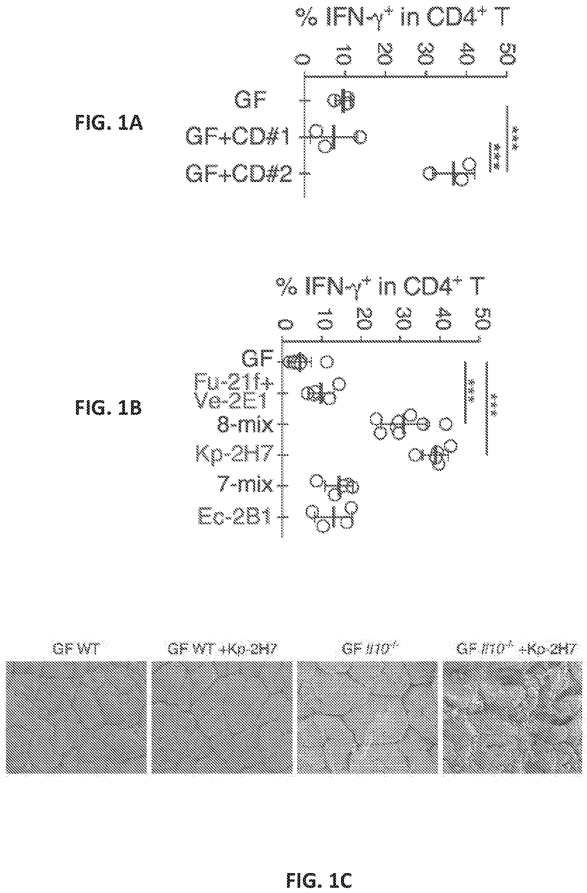

**Figs. 1A-C.** Isolation of TH1-cell-inducing, and proinflammatory *Klebsiella pneumoniae* strain from human saliva microbiota. **(****Fig. 1A****)** Frequencies of IFN-γ⁺ within colonic lamina propria (LP) CD4⁺TCRβ⁺ T cells from saliva microbiota-colonized germ-free (ex-GF) mice inoculated with saliva samples from patients with CD. Each point represents an individual mouse. (**Fig. 1B**) The percentage of TH1 cells in the colonic LP of B6 mice colonized with 8-mix, Veillo+Fuso, Kp-2H7, or 7-mix. (**Fig. 1C**) SEM images of the proximal colon of Kp-2H7- or Ec-2B 1-monocolonized WT or *Il10*^{*-*/*-*} mice. Symbols represent individual mice. Error bars indicate mean ± SEM. Data represent at least 2 independent experiments with similar results. ^{∗}*P* < 0.02; *^{∗∗}P* < 0.001.

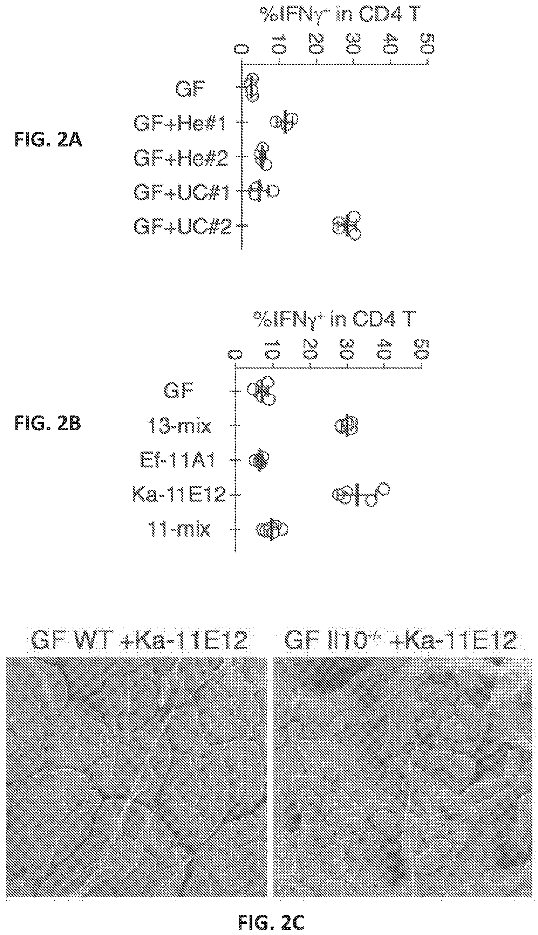

**Figs 2A-C.** *Enterobacter aerogenes* as another TH1-cell-inducing oral cavity bacterial species. (**Fig. 2A**) Frequencies of IFNγ⁺ within colonic LP CD4⁺TCRβ⁺ T cells from ex-GF mice inoculated with saliva samples from healthy donors and UC patients. Each point represents an individual mouse. (**Fig. 2B**) The percentage of TH1 cells in the colonic LP of B6 mice colonized with 13-mix, Efae-11A1, Ka-11E12, or 11-mix. (**Fig. 2C**) SEM images of the proximal colon of Ea-1 1E12-monocolonized WT or *Il10*^{*-*/*-*} mice.

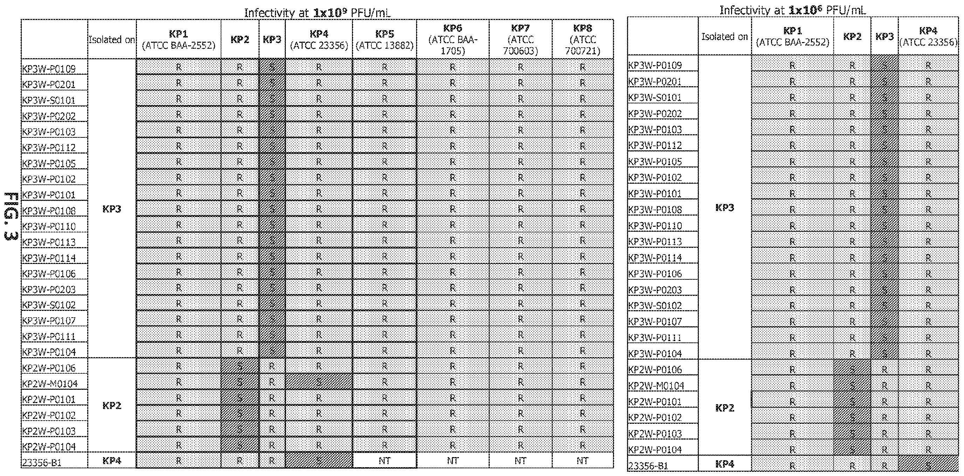

**Fig. 3****.** Host range analysis of phage isolated against KP2 and against KP3, including activity against other Klebsiella strains as indicated. Activity was measured at two phage titers - **1×10⁹** PFU/mL and **1×10⁶** PFU/mL. S- sensitive, R- resistant, NT- not tested, PFU - plaque forming units

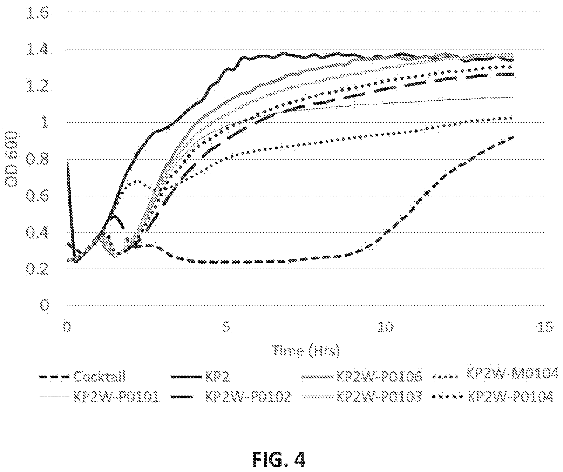

**Fig. 4****.** Appearance of phage resistant mutants in the KP2 culture infected with single phages and with a cocktail of 6 phage compared to the non-infected control. KP2 growth curve without phage is illustrated by the black line, while the remaining lines present the growth curves of KP2 with a single phage as indicated. The dashed line represents KP2 growth curve in the presence of the cocktail of six phages. The KP2 growth curves generated in the presence of individual phages demonstrate the regrowth of resistant or appearance of mutant strains after 1-2 hrs. In contrast, the growth of KP2 in the presence of the cocktail displays appearance of resistant mutants after 9 hrs enabling significant improvement in a "time to mutant" parameter. Similar analysis performed for KP3 strain resulted in tripled "time to mutant" or even no appearance of mutants during the course of the study.

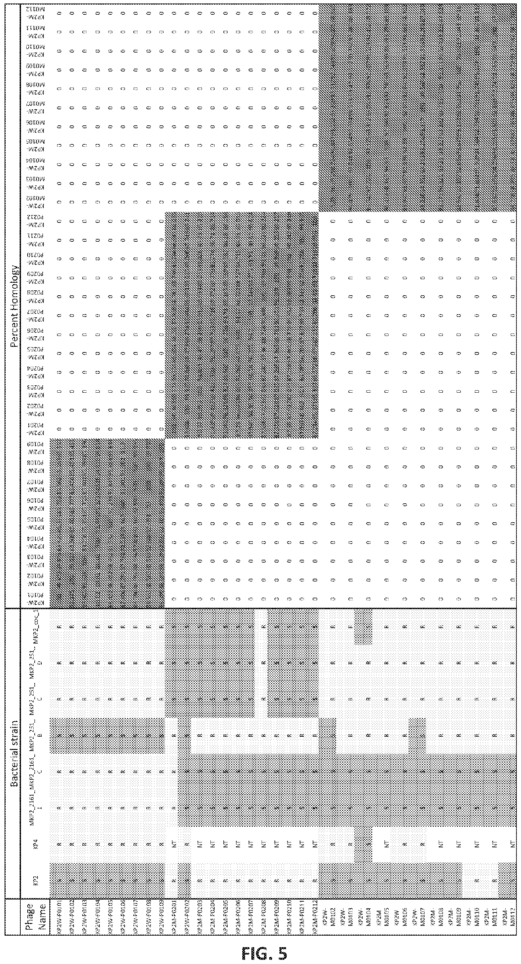

**Fig. 5****.** Host range analysis and percent homology of phages isolated against mutant KP2 strains resistant to the infection by the original KP2 phages. (Left) Host range analysis for phage isolated on the resistant bacterial mutant strain MKP2_2161_1 (resistant to phage KP2W-P0104) and MKP2_coc_1, a resistant bacterial mutant strain (resistant to phages KP2W-P0101, KP2W-P0102, KP2W-P0103, KP2W-P0104, KP2W-P0106, KP2W-M0104) and on KP2 strains, was performed on four additional KP mutants; MKP2_2161_C (resistant to the infection by KP2W-P0104), MKP2_251_B, MKP2_251_C and MKP2_251_D (resistant to the infection by KP2W-M0104). Each of the phage were added (10 µL) to bacterial lawns of the different K. pneumoniae strains, in 48 well plates by drop assay. Plates were incubated for 2-3 hrs (37°C) in aerobic conditions, after which plaques become visible on the bacterial lawns. Plates with bacterial lawns of MKP2_2161_1, MKP2_coc_1, KP2 and their respective phage served as positive control. Host range was done for each of the phage with two phage titers; 1×10⁶ PFU/mL and 1×10⁹ PFU/mL. R-resistant to the infection with the tested phage, S- sensitive to the infection with the tested phage, NT- not tested. However phage that were isolated on KP2 infected part or all KP2 mutant strains as well as the commercial strain KP4. (**Right**) Percent homology between the phages genomes is determined by combining all non-overlapping BLASTN alignment segments (BLAST HSPs), summing the values of their "Number of identical matches" and dividing this sum by the length of the query sequence. In some embodiments, this results in a non-symmetrical matrix.

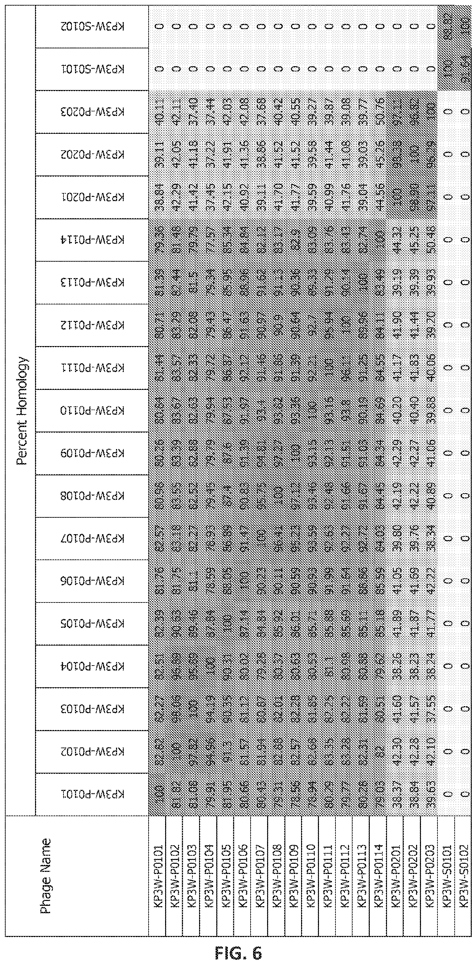

**Fig. 6****.** Percent homology of phages isolated against KP3, including 19 phage of class podoviridae (indicated by P) and 2 phage of class siphoviridae (Indicated by S). Percent homology between the phages genomes is determined by combining all non-overlapping BLASTN alignment segments (BLAST HSPs), summing the values of their "Number of identical matches" and dividing this sum by the length of the query sequence. In some embodiments, this results in a non-symmetrical matrix.

Sequences of exemplary bacteriophage and bacteria are disclosed in Table 1. The KP2 genome has been sequenced and is known in the art, *see, e.g.,* GenBank BDQR01000001.1 (https://www.ncbi.nlm.nih.gov/nuccore/BDQR01000001.1), GenBank GCA_002260905.1 (https://www.ncbi.nlm.nih.gov/assembly/GCA_002260905.1), BioSample: SAMD00083910 (https://www.ncbi.nlm.nih.gov/biosample/SAMD00083910/), BioProject PRJDB5883 (https://www.ncbi.nlm.nih.gov/bioproject/PRJDB5883), and Atarashi et al., Ectopic colonization of oral bacteria in the intestine drives TH1 cell induction and inflammation, Science (2017). In some embodiments, a genomic sequence, e.g., a KP2 genomic sequence, may encompass minor sequencing differences, e.g., less than 0.5%, less than 1%, less than 1.5%, less than 2%, less than 2.5%, or less than 3% as compared to another genomic sequence of the same bacterium, and the sequencing differences should not result in functional differences, e.g., bacteriophage infectivity, of the bacterium.

**Table 1. Brief Description of the Sequences**

| SEQ ID NO: | Sequence Name |
|---|---|
| 1 | KP3W-P0101 Bacteriophage Sequence |
| 2 | KP3W-P0102 Bacteriophage Sequence |
| 3 | KP3W-P0103 Bacteriophage Sequence |
| 4 | KP3W-P0104 Bacteriophage Sequence |
| 5 | KP3W-P0105 Bacteriophage Sequence |
| 6 | KP3W-P0106 Bacteriophage Sequence |
| 7 | KP3W-P0107 Bacteriophage Sequence |
| 8 | KP3W-P0108 Bacteriophage Sequence |
| 9 | KP3W-P0109 Bacteriophage Sequence |
| 10 | KP3W-P0110 Bacteriophage Sequence |
| 11 | KP3W-P0111 Bacteriophage Sequence |
| 12 | KP3W-P0112 Bacteriophage Sequence |
| 13 | KP3W-P0113 Bacteriophage Sequence |
| 14 | KP3W-P0114 Bacteriophage Sequence |
| 15 | KP3W-P0201 Bacteriophage Sequence |
| 16 | KP3W-P0202 Bacteriophage Sequence |
| 17 | KP3W-P0203 Bacteriophage Sequence |
| 18 | KP3W-S0101 Bacteriophage Sequence |
| 19 | KP3W-S0102 Bacteriophage Sequence |
| 20 | KP3W-S0102 Bacteriophage Sequence |
| 21 | KP2W-P0101 Bacteriophage Sequence |
| 22 | KP2W-P0102 Bacteriophage Sequence |
| 23 | KP2W-P0103 Bacteriophage Sequence |
| 24 | KP2W-P0104 Bacteriophage Sequence |
| 25 | KP2W-P0105 Bacteriophage Sequence |
| 26 | KP2W-P0106 Bacteriophage Sequence |
| 27 | KP2W-P0107 Bacteriophage Sequence |
| 28 | KP2W-P0108 Bacteriophage Sequence |
| 29 | KP2W-P0109 Bacteriophage Sequence |
| 30 | KP2W-M0102 Bacteriophage Sequence |
| 31 | KP2W-M0103 Bacteriophage Sequence |
| 32 | KP2W-M0104 Bacteriophage Sequence |
| 33 | KP2M-M0105 Bacteriophage Sequence |
| 34 | KP2W-M0106 Bacteriophage Sequence |
| 35 | KP2W-M0107 Bacteriophage Sequence |
| 36 | KP2M-M0108 Bacteriophage Sequence |
| 37 | KP2M-M0109 Bacteriophage Sequence |
| 38 | KP2M-M0110 Bacteriophage Sequence |
| 39 | KP2M-M0111 Bacteriophage Sequence |
| 40 | KP2M-M0112 Bacteriophage Sequence |
| 41 | KP2M-P0201 Bacteriophage Sequence |
| 42 | KP2W-P0202 Bacteriophage Sequence |
| 43 | KP2M-P0203 Bacteriophage Sequence |
| 44 | KP2M-P0204 Bacteriophage Sequence |
| 45 | KP2M-P0205 Bacteriophage Sequence |
| 46 | KP2M-P0206 Bacteriophage Sequence |
| 47 | KP2M-P0207 Bacteriophage Sequence |
| 48 | KP2M-P0208 Bacteriophage Sequence |
| 49 | KP2M-P0209 Bacteriophage Sequence |
| 50 | KP2M-P0210 Bacteriophage Sequence |
| 51 | KP2M-P0211 Bacteriophage Sequence |
| 52 | KP2M-P0212 Bacteriophage Sequence |
| 53 | MKP2_coc_1 Mutant Bacteria Sequence |
| 54 | MKP2_coc_1 Mutant Bacteria Sequence |
| 55 | MKP2_coc_1 Mutant Bacteria Sequence |
| 56 | MKP2_coc_1 Mutant Bacteria Sequence |
| 57 | MKP2_coc_1 Mutant Bacteria Sequence |
| 58 | MKP2_coc_1 Mutant Bacteria Sequence |
| 59 | MKP2_coc_1 Mutant Bacteria Sequence |
| 60 | MKP2_coc_1 Mutant Bacteria Sequence |
| 61 | MKP2_coc_1 Mutant Bacteria Sequence |
| 62 | MKP2_coc_1 Mutant Bacteria Sequence |
| 63 | MKP2_coc_1 Mutant Bacteria Sequence |
| 64 | MKP2_coc_1 Mutant Bacteria Sequence |
| 65 | MKP2_coc_1 Mutant Bacteria Sequence |
| 66 | MKP2_coc_1 Mutant Bacteria Sequence |
| 67 | MKP2_coc_1 Mutant Bacteria Sequence |
| 68 | MKP2_coc_1 Mutant Bacteria Sequence |
| 69 | MKP2_coc_1 Mutant Bacteria Sequence |
| 70 | MKP2_coc_1 Mutant Bacteria Sequence |
| 71 | MKP2_coc_1 Mutant Bacteria Sequence |
| 72 | MKP2_coc_1 Mutant Bacteria Sequence |
| 73 | MKP2_coc_1 Mutant Bacteria Sequence |
| 74 | MKP2_coc_1 Mutant Bacteria Sequence |
| 75 | MKP2_coc_1 Mutant Bacteria Sequence |
| 76 | MKP2_coc_1 Mutant Bacteria Sequence |
| 77 | MKP2_coc_1 Mutant Bacteria Sequence |
| 78 | MKP2_coc_1 Mutant Bacteria Sequence |
| 79 | MKP2_coc_1 Mutant Bacteria Sequence |
| 80 | MKP2_coc_1 Mutant Bacteria Sequence |
| 81 | MKP2_coc_1 Mutant Bacteria Sequence |
| 82 | MKP2_coc_1 Mutant Bacteria Sequence |
| 83 | MKP2_coc_1 Mutant Bacteria Sequence |
| 84 | MKP2_coc_1 Mutant Bacteria Sequence |
| 85 | MKP2_coc_1 Mutant Bacteria Sequence |
| 86 | MKP2_coc_1 Mutant Bacteria Sequence |
| 87 | MKP2_coc_1 Mutant Bacteria Sequence |
| 88 | MKP2_coc_1 Mutant Bacteria Sequence |
| 89 | MKP2_coc_1 Mutant Bacteria Sequence |
| 90 | MKP2_coc_1 Mutant Bacteria Sequence |
| 91 | MKP2_coc_1 Mutant Bacteria Sequence |
| 92 | MKP2_coc_1 Mutant Bacteria Sequence |
| 93 | MKP2_coc_1 Mutant Bacteria Sequence |
| 94 | MKP2_coc_1 Mutant Bacteria Sequence |
| 95 | MKP2_coc_1 Mutant Bacteria Sequence |
| 96 | MKP2_coc_1 Mutant Bacteria Sequence |
| 97 | MKP2_coc_1 Mutant Bacteria Sequence |
| 98 | MKP2_coc_1 Mutant Bacteria Sequence |
| 99 | MKP2_coc_1 Mutant Bacteria Sequence |
| 100 | MKP2_coc_1 Mutant Bacteria Sequence |
| 101 | MKP2_coc_1 Mutant Bacteria Sequence |
| 102 | MKP2_coc_1 Mutant Bacteria Sequence |
| 103 | MKP2_coc_1 Mutant Bacteria Sequence |
| 104 | MKP2_coc_1 Mutant Bacteria Sequence |
| 105 | MKP2_coc_1 Mutant Bacteria Sequence |
| 106 | MKP2_coc_1 Mutant Bacteria Sequence |
| 107 | MKP2_coc_1 Mutant Bacteria Sequence |
| 108 | MKP2_coc_1 Mutant Bacteria Sequence |
| 109 | MKP2_coc_1 Mutant Bacteria Sequence |
| 110 | MKP2_coc_1 Mutant Bacteria Sequence |
| 111 | MKP2_coc_1 Mutant Bacteria Sequence |
| 112 | MKP2_coc_1 Mutant Bacteria Sequence |
| 113 | MKP2_coc_1 Mutant Bacteria Sequence |
| 114 | MKP2_coc_1 Mutant Bacteria Sequence |
| 115 | MKP2_coc_1 Mutant Bacteria Sequence |
| 116 | MKP2_coc_1 Mutant Bacteria Sequence |
| 117 | MKP2_coc_1 Mutant Bacteria Sequence |
| 118 | MKP2_coc_1 Mutant Bacteria Sequence |
| 119 | MKP2_coc_1 Mutant Bacteria Sequence |
| 120 | MKP2_coc_1 Mutant Bacteria Sequence |
| 121 | MKP2_coc_1 Mutant Bacteria Sequence |
| 122 | MKP2_coc_1 Mutant Bacteria Sequence |
| 123 | MKP2_coc_1 Mutant Bacteria Sequence |
| 124 | MKP2_coc_1 Mutant Bacteria Sequence |
| 125 | MKP2_coc_1 Mutant Bacteria Sequence |
| 126 | MKP2_coc_1 Mutant Bacteria Sequence |
| 127 | MKP2_2161_1 Mutant Bacteria Sequence |
| 128 | MKP2_2161_1 Mutant Bacteria Sequence |
| 129 | MKP2_2161_1 Mutant Bacteria Sequence |
| 130 | MKP2_2161_1 Mutant Bacteria Sequence |
| 131 | MKP2_2161_1 Mutant Bacteria Sequence |
| 132 | MKP2_2161_1 Mutant Bacteria Sequence |
| 133 | MKP2_2161_1 Mutant Bacteria Sequence |
| 134 | MKP2_2161_1 Mutant Bacteria Sequence |
| 135 | MKP2_2161_1 Mutant Bacteria Sequence |
| 136 | MKP2_2161_1 Mutant Bacteria Sequence |
| 137 | MKP2_2161_1 Mutant Bacteria Sequence |
| 138 | MKP2_2161_1 Mutant Bacteria Sequence |
| 139 | MKP2_2161_1 Mutant Bacteria Sequence |
| 140 | MKP2_2161_1 Mutant Bacteria Sequence |
| 141 | MKP2_2161_1 Mutant Bacteria Sequence |
| 142 | MKP2_2161_1 Mutant Bacteria Sequence |
| 143 | MKP2_2161_1 Mutant Bacteria Sequence |
| 144 | MKP2_2161_1 Mutant Bacteria Sequence |
| 145 | MKP2_2161_1 Mutant Bacteria Sequence |
| 146 | MKP2_2161_1 Mutant Bacteria Sequence |
| 147 | MKP2_2161_1 Mutant Bacteria Sequence |
| 148 | MKP2_2161_1 Mutant Bacteria Sequence |
| 149 | MKP2_2161_1 Mutant Bacteria Sequence |
| 150 | MKP2_2161_1 Mutant Bacteria Sequence |
| 151 | MKP2_2161_1 Mutant Bacteria Sequence |
| 152 | MKP2_2161_1 Mutant Bacteria Sequence |
| 153 | MKP2_2161_1 Mutant Bacteria Sequence |
| 154 | MKP2_2161_1 Mutant Bacteria Sequence |
| 155 | MKP2_2161_1 Mutant Bacteria Sequence |
| 156 | MKP2_2161_1 Mutant Bacteria Sequence |
| 157 | MKP2_2161_1 Mutant Bacteria Sequence |
| 158 | MKP2_2161_1 Mutant Bacteria Sequence |
| 159 | MKP2_2161_1 Mutant Bacteria Sequence |
| 160 | MKP2_2161_1 Mutant Bacteria Sequence |
| 161 | MKP2_2161_1 Mutant Bacteria Sequence |
| 162 | MKP2_2161_1 Mutant Bacteria Sequence |
| 163 | MKP2_2161_1 Mutant Bacteria Sequence |
| 164 | MKP2_2161_1 Mutant Bacteria Sequence |
| 165 | MKP2_2161_1 Mutant Bacteria Sequence |
| 166 | MKP2_2161_1 Mutant Bacteria Sequence |
| 167 | MKP2_2161_1 Mutant Bacteria Sequence |
| 168 | MKP2_2161_1 Mutant Bacteria Sequence |
| 169 | MKP2_2161_1 Mutant Bacteria Sequence |
| 170 | MKP2_2161_1 Mutant Bacteria Sequence |
| 171 | MKP2_2161_1 Mutant Bacteria Sequence |
| 172 | MKP2_2161_1 Mutant Bacteria Sequence |
| 173 | MKP2_2161_1 Mutant Bacteria Sequence |
| 174 | MKP2_2161_1 Mutant Bacteria Sequence |
| 175 | MKP2_2161_1 Mutant Bacteria Sequence |
| 176 | MKP2_2161_1 Mutant Bacteria Sequence |
| 177 | MKP2_2161_1 Mutant Bacteria Sequence |
| 178 | MKP2_2161_1 Mutant Bacteria Sequence |
| 179 | MKP2_2161_1 Mutant Bacteria Sequence |
| 180 | MKP2_2161_1 Mutant Bacteria Sequence |
| 181 | MKP2_2161_1 Mutant Bacteria Sequence |
| 182 | MKP2_2161_1 Mutant Bacteria Sequence |
| 183 | MKP2_2161_1 Mutant Bacteria Sequence |
| 184 | MKP2_2161_1 Mutant Bacteria Sequence |
| 185 | MKP2_2161_1 Mutant Bacteria Sequence |
| 186 | MKP2_2161_1 Mutant Bacteria Sequence |
| 187 | MKP2_2161_1 Mutant Bacteria Sequence |
| 188 | MKP2_2161_1 Mutant Bacteria Sequence |
| 189 | MKP2_2161_1 Mutant Bacteria Sequence |
| 190 | MKP2_2161_1 Mutant Bacteria Sequence |
| 191 | MKP2_2161_1 Mutant Bacteria Sequence |
| 192 | MKP2_2161_1 Mutant Bacteria Sequence |
| 193 | MKP2_2161_1 Mutant Bacteria Sequence |
| 194 | MKP2_2161_1 Mutant Bacteria Sequence |
| 195 | MKP2_2161_1 Mutant Bacteria Sequence |
| 196 | MKP2_2161_1 Mutant Bacteria Sequence |
| 197 | MKP2_2161_1 Mutant Bacteria Sequence |
| 198 | MKP2_2161_1 Mutant Bacteria Sequence |
| 199 | MKP2_2161_1 Mutant Bacteria Sequence |
| 200 | MKP2_2161_1 Mutant Bacteria Sequence |
| 201 | MKP2_2161_1 Mutant Bacteria Sequence |
| 202 | MKP2_2161_1 Mutant Bacteria Sequence |
| 203 | MKP2_2161_1 Mutant Bacteria Sequence |
| 204 | MKP2_2161_1 Mutant Bacteria Sequence |
| 205 | MKP2_2161_1 Mutant Bacteria Sequence |
| 206 | MKP2_2161_1 Mutant Bacteria Sequence |
| 207 | MKP2_2161_1 Mutant Bacteria Sequence |
| 208 | MKP2_2161_1 Mutant Bacteria Sequence |
| 209 | MKP2_2161_1 Mutant Bacteria Sequence |
| 210 | MKP2_2161_1 Mutant Bacteria Sequence |
| 211 | MKP2_2161_1 Mutant Bacteria Sequence |
| 212 | MKP2_2161_1 Mutant Bacteria Sequence |
| 213 | MKP2_2161_1 Mutant Bacteria Sequence |
| 214 | MKP2_2161_1 Mutant Bacteria Sequence |
| 215 | MKP2_2161_1 Mutant Bacteria Sequence |
| 216 | MKP2_2161_1 Mutant Bacteria Sequence |
| 217 | MKP2_2161_1 Mutant Bacteria Sequence |
| 218 | MKP2_2161_1 Mutant Bacteria Sequence |
| 219 | MKP2_2161_1 Mutant Bacteria Sequence |
| 220 | MKP2_2161_1 Mutant Bacteria Sequence |
| 221 | MKP2_2161_1 Mutant Bacteria Sequence |
| 222 | MKP2_2161_1 Mutant Bacteria Sequence |
| 223 | MKP2_2161_1 Mutant Bacteria Sequence |
| 224 | MKP2_2161_1 Mutant Bacteria Sequence |
| 225 | MKP2_2161_1 Mutant Bacteria Sequence |
| 226 | MKP2_2161_1 Mutant Bacteria Sequence |
| 227 | MKP2_2161_1 Mutant Bacteria Sequence |
| 228 | MKP2_2161_1 Mutant Bacteria Sequence |
| 229 | MKP2_2161_1 Mutant Bacteria Sequence |
| 230 | MKP2_2161_1 Mutant Bacteria Sequence |
| 231 | MKP2_2161_1 Mutant Bacteria Sequence |
| 232 | MKP2_2161_1 Mutant Bacteria Sequence |
| 233 | MKP2_2161_1 Mutant Bacteria Sequence |
| 234 | MKP2_2161_1 Mutant Bacteria Sequence |
| 235 | MKP2_2161_1 Mutant Bacteria Sequence |
| 236 | MKP2_2161_1 Mutant Bacteria Sequence |
| 237 | MKP2_2161_1 Mutant Bacteria Sequence |
| 238 | MKP2_2161_1 Mutant Bacteria Sequence |
| 239 | MKP2_2161_1 Mutant Bacteria Sequence |
| 240 | MKP2_2161_1 Mutant Bacteria Sequence |
| 241 | MKP2_2161_1 Mutant Bacteria Sequence |
| 242 | MKP2_2161_1 Mutant Bacteria Sequence |
| 243 | MKP2_2161_1 Mutant Bacteria Sequence |
| 244 | MKP2_2161_1 Mutant Bacteria Sequence |
| 245 | MKP2_2161_1 Mutant Bacteria Sequence |
| 246 | MKP2_2161_1 Mutant Bacteria Sequence |
| 247 | MKP2_2161_1 Mutant Bacteria Sequence |
| 248 | MKP2_2161_1 Mutant Bacteria Sequence |
| 249 | MKP2_2161_1 Mutant Bacteria Sequence |
| 250 | MKP2_2161_1 Mutant Bacteria Sequence |
| 251 | MKP2_2161_1 Mutant Bacteria Sequence |
| 252 | MKP2_2161_1 Mutant Bacteria Sequence |
| 253 | MKP2_2161_1 Mutant Bacteria Sequence |
| 254 | MKP2_2161_1 Mutant Bacteria Sequence |
| 255 | MKP2_2161_1 Mutant Bacteria Sequence |
| 256 | MKP2_2161_1 Mutant Bacteria Sequence |
| 257 | MKP2_2161_1 Mutant Bacteria Sequence |
| 258 | MKP2_2161_1 Mutant Bacteria Sequence |
| 259 | MKP2_2161_1 Mutant Bacteria Sequence |
| 260 | MKP2_2161_1 Mutant Bacteria Sequence |
| 261 | MKP2_2161_1 Mutant Bacteria Sequence |
| 262 | MKP2_2161_1 Mutant Bacteria Sequence |
| 263 | MKP2_2161_1 Mutant Bacteria Sequence |
| 264 | MKP2_2161_1 Mutant Bacteria Sequence |
| 265 | MKP2_2161_1 Mutant Bacteria Sequence |
| 266 | MKP2_2161_1 Mutant Bacteria Sequence |
| 267 | MKP2_2161_1 Mutant Bacteria Sequence |
| 268 | MKP2_2161_1 Mutant Bacteria Sequence |
| 269 | MKP2_2161_1 Mutant Bacteria Sequence |
| 270 | MKP2_2161_1 Mutant Bacteria Sequence |
| 271 | MKP2_2161_1 Mutant Bacteria Sequence |
| 272 | MKP2_2161_1 Mutant Bacteria Sequence |
| 273 | MKP2_2161_1 Mutant Bacteria Sequence |
| 274 | MKP2_2161_1 Mutant Bacteria Sequence |
| 275 | MKP2_2161_1 Mutant Bacteria Sequence |
| 276 | MKP2_2161_1 Mutant Bacteria Sequence |
| 277 | MKP2_2161_1 Mutant Bacteria Sequence |
| 278 | MKP2_2161_1 Mutant Bacteria Sequence |
| 279 | MKP2_2161_1 Mutant Bacteria Sequence |
| 280 | MKP2_2161_1 Mutant Bacteria Sequence |
| 281 | MKP2_2161_1 Mutant Bacteria Sequence |
| 282 | MKP2_2161_1 Mutant Bacteria Sequence |
| 283 | KP2 Bacteria Sequence |
| 284 | KP2 Bacteria Sequence |
| 285 | KP2 Bacteria Sequence |
| 286 | KP3 Bacteria Sequence |

### Description of Embodiments

The disclosure relates to bacteriophage, pharmaceutical compositions thereof, methods of modulating IBD (Jostins et al., 2012), and methods of selecting patients that are responsive to treatment by the methods set forth herein. In some embodiments, the bacteriophage described herein is capable of lysing *Klebsiella* bacterial that are associated with IBD. *Klebisella pneumoniae* have been linked to IBD. IBD patients demonstrate elevated systemic antibodies against certain capsular types (e.g., K2, K17, K26, K36, K50, and K21 found in KP2) compared to controls and other pathologic conditions. These findings suggest the involvement of *Klebsiella* in IBD, but do not explain whether *Klebsiella* is actively involved in the pathogenesis of the disease and do not provide strain-specific resolution as different strains share similar capsular types. Example 1 shows the relationship between intestinal administration of a single pathobiont strain (KP2 or KP3) and an immune response in the intestine (TH1 polarization in the lamina propria) and induction of colitis *in vivo* (in I110-/- mice).

### Definitions

In order that the disclosure may be more readily understood, certain terms are first defined. These definitions should be read in light of the remainder of the disclosure and as understood by a person of ordinary skill in the art. Unless defined otherwise, all technical and scientific terms used herein have the same meaning as commonly understood by a person of ordinary skill in the art. Additional definitions are set forth throughout the detailed description.

As used herein, the terms "treat" and "modulate" and their cognates refer to an amelioration of IBD or at least one discernible symptom thereof. In some embodiments, "treat" and "modulate" and their cognates refer to an amelioration of at least one measurable physical parameter, not necessarily discernible by the patient. In some embodiments, "treat" and "modulate" and their cognates refer to inhibiting or reducing or slowing the progression of IBD, either physically (e.g., stabilization of a discernible symptom), physiologically (e.g., stabilization of a physical parameter), or both, relative to an untreated control. In certain embodiments, "treat" and "modulate" and their cognates refer to slowing the progression or reversing the progression of IBD relative to an untreated control. As used herein, "prevent" and its cognates refer to delaying the onset or reducing the risk of acquiring IBD or a symptom associated with IBD relative to an untreated control.

In certain embodiments, the bacteriophage described herein is administered to ameliorate an IBD in a subject and results in one or more symptoms or physical parameters of the condition or disorder to improve by at least about 10%, 20%, 30%, 40%, 50%, 60%, 70%, 80%, 90%, 95%, or more as compared to levels in an untreated or control subject. In some embodiments, the improvement is measured by comparing the symptom or physical parameter in a subject before and after administration of the bacteriophage. In some embodiments, the measurable physical parameter is a reduction in bacterial CFU in the stool.

The measurable physical parameter may be any suitable clinical parameter known in the art, e.g., for Crohn's disease (Sandborn et al., 2013; Best et al., 1976; Warrel - Oxford Textbook of Medicine, 5th edition) or for ulcerative colitis (Tursi et al., 2010; Warrel - Oxford Textbook of Medicine, 5th edition). Crohn's disease parameters include but are not limited to assessment of clinical remission using Crohn's Disease Activity Index (CDAI) (CDAI score of ≤150 points) and CDAI-100 response (≥100-point decrease in the CDAI score from the baseline); inflammation reduction as measured by decrease in serum levels of C-reactive protein, differential blood count and serum albumin levels; reduced levels of fecal calprotectin and improvement in the appearance of the mucosal layer in terminal ileum and rectum as evaluated by microscopic examination of the biopsies collected during endoscopic procedures (e.g., reduction in neutrophil, macrophage and other immune cell infiltration, granuloma size and number, reduction in the number and size of ulcers, edematous mucosa). In addition the improvement of the disease may be evaluated based on the assessment of the clinical symptoms including diarrhea, abdominal pain, weight loss, fever, vomiting, colic, rectal bleeding, anemia, extraintestinal manifestations (hypersensitivity rash to sulphasalazine, erythema nodosum, pyoderma gangrenosum, oral aphthous ulcers, sore tongue, angular stomatitis, episcleritis or an anterior uveitis, arthritis, acute arthropathy, low back pain, sacroiliitis, ankylosing spondylitis, liver diseases: minor elevations of alkaline phosphatase or transaminases, primary sclerosing cholangitis, chronic liver disease ranging from an autoimmune hepatitis to the classic picture of concentric periductular fibrosis with obliteration of bile ducts, pericarditis with or without an effusion, autoimmune haemolytic anaemia, amyloid, rapidly progressing bronchiectasis).

Ulcerative colitis disease parameters include but not limited to improvement in the ulcerative colitis disease activity index (UCDAI) (UCDAI decrease of 50% or more from the baseline), activity of relapsing UC; remission, considered as UCDAI ≤ 2; improvement in hemoglobin levels, serum albumin levels, decrease in C-reactive protein levels; improvement in neutrophil leukocytosis (blood differential count); improvement in endoscopic scores (reduction in the edematous mucosa area, reduction in neutrophil, macrophage and other immune cell infiltration, reduction in the number and size of ulcers, edematous mucosa; improvement in the clinical symptoms: rectal bleeding, fever, stool frequency, diarrhea, the passage of mucus, abdominal pain, constipation, anorexia, nausea, weight loss, malaise, lassitude, symptoms of chronic iron deficiency, minor perianal disease, such as a fissure, extraintestinal manifestations described above, and additionally oral candidiasis, finger clubbing.

Those in need of treatment may include individuals already having IBD, as well as those at risk of having, or who may ultimately acquire the disease. The need for treatment is assessed, e.g., by the presence of one or more risk factors associated with the development of IBD, the presence or progression of IBD, or likely receptiveness to treatment of a subject having IBD. For example, "treating" IBD may encompass reducing or eliminating associated symptoms, and does not necessarily encompass the elimination of the underlying disease etiology, e.g., a genetic instability locus.

In some embodiments, an individual with IBD is in remission and/or presently asymptomatic, and the bacteriophage described herein may be administered during the remission period to reduce the potential for a flare-up. In some embodiments, the individual in remission and/or presently asymptomatic for IBD is undergoing treatment, e.g., antibiotics and/or steroids, and the bacteriophage described herein may be co-administered with such treatment to reduce the potential for a flare-up.

*Klebsiella* is a genus of bacteria belonging to the Enterobacteriaceae family. *Klebsiella* are gram-negative, nonmotile, and rod-shaped. In some embodiments, the species of *Klebsiella* may be associated with human disease, e.g., *Klebsiella pneumoniae.* As used herein, *Klebsiella* includes bacteria that are currently classified, were previously classified, or will be reclassified as *Klebsiella* bacteria, e.g., *Klebsiella aeromobilis,* which is also known as *Enterobacter aerogenes* (Tindall et al., 2017; Diene et al., 2013). In some embodiments, *Klebsiella* refers to *Klebsiella pneumoniae.* In some embodiments, *Klebsiella* refers to *Klebsiella aeromobilis*/*Enterobacter aerogenes* bacteria. In some embodiments, *Klebsiella* refers to naturally occurring *Klebsiella.* In some embodiments, *Klebsiella* refers to naturally occurring, variant or mutant *Klebsiella* (e.g., antibiotic resistant, phage resistant, nosocomial). In some embodiments, the variant or mutant *Klebsiella* is resistant to at least 1, at least 2, at least 3, at least 4, or at least 5 antibiotics. In some embodiments, a mutant bacterial strain may arise in the presence of a bacteriophage and become resistant to said bacteriophage.

As used herein, a "strain" of bacteria refers to a genetic variant or subtype of bacteria. In some embodiments, a "strain" of bacteria comprises descendants from a single isolation in a pure culture of said bacteria. As used herein, a "strain" of bacteria may refer to one or more genetic variants or subtypes of said bacteria. For example, as used herein, a "strain" of *Klebsiella pneumoniae* may refer to one or more genetic variants or subtypes of *Klebsiella pneumoniae,* including but not limited to KP1 (ATCC BAA-2552; also referred to as *Klebsiella variicola*), KP2 (*Klebsiella pneumoniae* strain 2H7 described herein), KP4 (ATCC 23356), KP5 (ATCC 13882), KP6 (ATCC BAA-1705), KP7 (ATCC 700603), and KP8 (ATCC 700721). Similarly, as used herein, a bacteriophage that is capable of lysing a "strain" of *Klebsiella pneumoniae* refers to a bacteriophage that is capable of lysing one or more genetic variants or subtypes of *Klebsiella pneumoniae,* including but not limited to KP1, KP2, KP3, KP4, KP5, KP6, KP7, and KP8. In some embodiments, a bacteriophage that is capable of lysing a "strain" of *Klebsiella pneumoniae* refers to a bacteriophage that is capable of lysing one or more genetic variants or subtypes of *Klebsiella pneumoniae* KP1, KP2, KP4, KP5, KP6, KP7, and KP8. In some embodiments, a bacteriophage that is capable of lysing a "strain" of *Klebsiella pneumoniae* refers to a bacteriophage that is capable of lysing one or more genetic variants or subtypes of *Klebsiella pneumoniae* KP2, KP4, KP5, KP6, KP7, and KP8. In some embodiments, a bacteriophage that is capable of lysing a "strain" of *Klebsiella aeromobilis*/*Enterobacter aerogenes* refers to a bacteriophage that is capable of lysing one or more genetic variants or subtypes of *Klebsiella aeromobilis*/*Enterobacter aerogenes* KP3, e.g., strain 11E12 described herein. In some embodiments, the KP2 bacteriophage described herein is capable of lysing a KP2 strain of *Klebsiella pneumoniae.* In some embodiments, the KP3 bacteriophage described herein is capable of lysing a KP3 strain of *Klebsiella aeromobilis.*

In some embodiments, as used herein, a "mutant" bacterium refers to a bacterium that comprises greater than about 85%, greater than about 90%, greater than about 95%, greater than about 97%, or greater than about 99% homology to a corresponding wild-type bacterial strain. For example, a mutant KP2 bacterium comprises greater than about 85%, greater than about 90%, greater than about 95%, greater than about 97%, or greater than about 99% homology to a wild-type KP2 bacterium (Table 1, contigs). A mutant KP3 bacterium comprises greater than about 85%, greater than about 90%, greater than about 95%, greater than about 97%, or greater than about 99% homology to a wild-type KP3 bacterium (Table 1).

In some embodiments, a "mutant" KP2 bacterium is a bacterium that is capable of being lysed by the KP2 bacteriophage disclosed herein. In some embodiments, a "mutant" KP2 bacterium refers to a bacterium comprising less than about 85% homology as compared to wild-type KP2, but is capable of being lysed by the KP2 bacteriophage disclosed herein. In some embodiments, a "mutant" KP3 bacterium is a bacterium that is capable of being lysed by the KP3 bacteriophage disclosed herein. In some embodiments, a "mutant" KP3 bacterium refers to a bacterium comprising less than about 85% homology as compared to wild-type KP3, but is capable of being lysed by the KP3 bacteriophage disclosed herein.

In some embodiments, a KP2 bacterium, e.g., a strain of KP2 bacterium, and/or a mutant KP2 bacterium, e.g., an environmental and/or clinical isolate as described herein, is positive for a unique genomic region that may be identified by PCR using the following primers: 5'AGCACTAGCGGCTGTGGTAT3' and 5'ACTTACTCGGGCCCTTGATT3'. *See, e.g.,* Atarashi et al., 2017. In some embodiments, the mutant KP2 bacterium, e.g., an environmental and/or clinical isolate identified using said primers, comprises greater than about 95%, greater than about 96%, greater than about 97%, greater than about 98%, or greater than about 99% homology to the KP2 bacterium (Table 1, contigs), e.g., % homology of the coding regions as calculated by ANI (Average Nucleotide Identity). *See, e.g.,* Han et al., ANItools web: a web tool for fast genome comparison within multiple bacterial strains (2016). In some embodiments, a mutant KP2 bacterium identified by said primers and is at least about 99% homologous to the KP2 bacterium (Table 1, contigs) is referred to as "KP2-like" herein. In some embodiments, the mutant and/or KP2-like bacterium is capable of being lysed by at least one of the KP2 bacteriophage disclosed herein.

As used herein, "bacteriophage" and "phage" are used interchangeably and refer to an isolated virus that is capable of infecting a bacterium. In some embodiments, the phage comprises a DNA or an RNA genome. A phage may be isolated from a natural or human-made environment. In some embodiments, the phage is selected from Myoviridae, Podoviridae, and Siphoviridae. As used herein, a "KP bacteriophage" is intended to refer to a bacteriophage that is capable of lysing a *Klebsiella pneumoniae* or *Klebsiella aeromobilis*/*Enterobacter aerogenes* bacterium. For example, a "KP2 bacteriophage" refers to a bacteriophage that is capable of lysing a *Klebsiella pneumoniae* strain KP2 bacterium, including wild-type and mutant KP2 bacterium. A "KP3 bacteriophage" refers to a bacteriophage that is capable of lysing a *Klebsiella aeromobilis*/*Enterobacter aerogenes* strain KP3 bacterium, including wild-type and mutant KP3 bacterium.

It is known that different isolates of a given bacteriophage may vary at the nucleic acid sequence level. In some embodiments, bacteriophages are considered to be "functionally equivalent" as long as they exhibit similar phenotypes, e.g., similar host range, similar lytic ability, and/or threshold sequence similarity (e.g., greater than about 85%, greater than about 90%, greater than about 95%, greater than about 97%, or greater than about 99%). As used herein, the term "bacteriophage" encompasses a parent bacteriophage as well as its progeny or derivatives.

As used herein, "host range" refers to the bacteria that are susceptible to infection by a particular phage. The host range of a phage may include, but is not limited to, a strain, a species, a genus, or multiple genera of bacteria. The term encompasses phage adsorbable, non-productive infection (e.g., restrictive, abortive, lysogenic), and productive infections. In some embodiments, a phage may recognize two or more strains. In some embodiments, a phage may recognize wild-type and phage-resistant mutant strains.

Different phage isolates may be prepared and phenotyped using methods known in the art, e.g., a plaque assay, liquid media assay, solid media assay. In some embodiments, the solid media assays to quantify and isolate phage are based on plaque assays (Abedon & Yin, 2009), ranging from efficiency of plating (EOP) (Kutter, 2009) to spot testing (Hyman & Abedon, 2010). In some embodiments, the plate format used for the plaque assay can be modified, e.g., from a petri dish to a 48-well plate.

In some embodiments, a double-layer plaque assay is used to phenotype bacteriophage isolates. For example, a starter culture of 4 mL BHIS may be inoculated with 5-10 colonies from a plate. This culture may be incubated at 37°C for 1.5-2 hours. A volume of 100 µL of this culture may be mixed with 100 µL of a phage-containing sample (or medium only control) and incubated for 15 minutes. Thereafter, 3 mL of BHIS top agar (pre-molten 0.4% agar BHIS supplemented with 1 mM Ca²⁺ and Mg²⁺ ions) may be added, and the mixture may be poured over a BHIS bottom agar plate (1.5% agar BHIS). The plates may be allowed to gel at room temperature, and then incubated for 2-3 hours at 37°C until plaques are identified.

In some embodiments, a modified spot drop assay is used to phenotype bacteriophage isolates. For example, a starter culture of 4 mL BHIS may be inoculated with 5-10 colonies from a plate. This culture may be incubated at 37°C for 1.5-2 hours. A volume of 100 µL of this culture may be mixed with 3 mL of BHIS top agar (pre-molten 0.4% agar BHIS supplemented with 1 mM Ca²⁺ and Mg²⁺ ions), and 100 µL of this mixture may be dispensed per well of a Nunc 48-well plate already containing 1 mL per well of BHIS bottom agar. After solidifying at room temperature, the plates may be incubated for 30 minutes at 37 degrees. At this stage, 10 µL of samples containing phage or media only controls may be dropped in the middle of the well, left to absorb, and then may be incubated for 2-3 hours until plaques are visible for counting.

In some embodiments, a liquid media assay is used to phenotype the bacteriophage. In some embodiments, liquid-based phage infection assays follow the time-course of infection and can provide more than quantitative end-points of infection as compared to the solid-phase plaque assays. In some embodiments, by mixing phage with bacteria in liquid medium, then following the turbidity of the culture over time, one can discern finer differences (e.g., a delay in the time of cell lysis) between how different bacterial strains interact with the phage. In some embodiments, the liquid media assay allows for high-throughput measurements by using 96-well plates and reading optical density in a plate reader.

For example, a bacterial strain may be grown for 1.5-2 hours until an OD₆₀₀ of about 1.7-2. This culture may then be diluted using BHIS medium to a starting optical density, typically between 0.05 and 0.2 OD₆₀₀. A volume of 200 µL of culture may then be dispensed into the wells of a Nunclon flat-bottomed 96-well plate. 10 µL of a sample containing phage or 10 µL of medium as control may be added to each well. The wells may be covered with 50 µL of mineral oil to limit evaporation, and a thin sterile optically transparent polyester film may be added to keep the culture sterile. Optical density measurements may be carried out every 15 minutes, e.g., in a Tecan Infinite M200 plate reader connected to a Tecan EVO75 robot. Between measurements, the plate may be incubated while shaking at 37°C, e.g., inside the EVO75 incubator.

In some embodiments, infectivity is determined by the plaque presence in a solid assay only. In some embodiments, infectivity is determined by the decrease in the bacterial culture optical density in a liquid assay only. In some embodiments, infectivity is determined by the decrease in the bacterial culture optical density and plaque presence in both the liquid assay and the solid assay.

As used herein, a "lytic" bacteriophage refers to a virulent bacteriophage that in the course attaches to a bacterial host and inserts its genetic material into the bacterial host cell. After that a phage usually follows one of two life cycles, lytic (virulent) or lysogenic (temperate). Lytic phages take over the machinery of the cell to make phage components. They then destroy, or lyse, the cell, releasing new phage particles. Lysogenic phages incorporate their nucleic acid into the chromosome of the host cell and replicate with it as a unit without destroying the cell. Under certain conditions lysogenic phages can be induced to follow a lytic cycle. In some embodiments, following the infection, new bacteriophage particles are released. In some embodiments, following the infection, the host bacterial cells are lysed and destroyed. In some embodiments, less than 90% of the host bacterial cells are lysed and destroyed. *See, e.g.,* Abedon et al., 2011; Sulakvelidze et al., 2001; Green et al., 2017.

In some embodiments, the % lysis is measured by methods known in the art and described herein, e.g., by optical density (OD) or qPCR.

As used herein, "% homology" refers to the level of nucleic acid sequence identity or amino acid sequence identity between a first nucleic acid or amino acid sequence when aligned to a second nucleic acid or amino acid sequence using a sequence alignment program. When a position in the first and the second sequences is occupied by the same nucleic acid or amino acid (e.g., if a position in the first nucleic acid sequence and the second nucleic acid sequence is occupied by cytosine), then the first and the second sequences are homologous at that position.

In general, homology between two sequences is calculated from the number of matching or homologous positions shared by the two sequences over the total number of positions compared. In some embodiments, the first and the second sequences are aligned in a manner to maximize % homology. In some embodiments, % homology refers to the % identity over the shorter of two sequences. In some embodiments, the % homology for a nucleic acid sequence includes intronic and/or intergenic regions. Exemplary levels of % homology include, but are not limited to, 80, 81, 82, 83, 84, 85, 86, 87, 88, 89, 90, 91, 92, 93, 94, 95, 96, 97, 98, 99% or more sequence identity between a first and a second sequence.

Exemplary sequence alignment programs that may be used to determine % homology between two sequences include, but are not limited to, the FASTA package (including rigorous (SSEARCH, LALIGN, GGSEARCH and GLSEARCH) and heuristic (FASTA, FASTX/Y, TFASTX/Y and FASTS/M/F) algorithms, the EMBOSS package (Needle, stretcher, water and matcher), the BLAST programs (including, but not limited to BLASTN, BLASTX, TBLASTX, BLASTP, TBLASTN), megablast and BLAT. In some embodiments, the sequence alignment program is BLASTN. For example, 95% homology refers to 95% sequence identity determined by BLASTN, by combining all non-overlapping alignment segments (BLAST HSPs), summing their numbers of identical matches and dividing this sum with the length of the shorter sequence.

In some embodiments, the sequence alignment program is a basic local alignment program, e.g., BLAST. In some embodiments, the sequence alignment program is a pairwise global alignment program. In some embodiments, the pairwise global alignment program is used for protein-protein alignments. In some embodiments, the pairwise global alignment program is Needle. In some embodiments, the sequence alignment program is a multiple alignment program. In some embodiments, the multiple alignment program is MAFFT. In some embodiments, the sequence alignment program is a whole genome alignment program. In some embodiments, the whole genome alignment is performed using BLASTN. In some embodiments, BLASTN is utilized without any changes to the default parameters.

As used herein, a "pharmaceutical composition" refers to a preparation of the bacteriophage of the invention with other components such as a physiologically suitable carrier and/or excipient.

"Physiologically acceptable carrier" and "pharmaceutically acceptable carrier" are used interchangeably herein to refer to a carrier or a diluent that does not cause significant irritation to an organism and does not abrogate the biological activity and properties of the administered bacteriophage composition. An adjuvant is included under these phrases.

The term "excipient" refers to an inert substance added to a pharmaceutical composition to further facilitate administration of an active ingredient. Examples include, but are not limited to, calcium bicarbonate, calcium phosphate, various sugars and types of starch, cellulose derivatives, gelatin, vegetable oils, polyethylene glycols, and surfactants, including, e.g., polysorbate 20.

The terms "therapeutically effective dose" and "therapeutically effective amount" are used to refer to an amount of a compound that results in prevention, delay of onset of symptoms, or amelioration of symptoms of IBD compared to an untreated control. A therapeutically effective amount may, for example, be sufficient to treat, prevent, reduce the severity, delay the onset, and/or reduce the risk of occurrence of one or more symptoms of IBD compared to an untreated control.

As used herein, "intestine" refers to the gastrointestinal tract of a mammal and encompasses the organs, glands, tracts, and systems that are responsible for the transfer and digestion of food, absorption of nutrients, and excretion of waste. In humans, the "intestine" comprises the gastrointestinal tract, which starts at the mouth and ends at the anus, and additionally comprises the esophagus, stomach, small intestine, and large intestine. The upper gastrointestinal tract comprises the esophagus, stomach, and duodenum of the small intestine. The lower gastrointestinal tract comprises the remainder of the small intestine, i.e., the jejunum and ileum, and all of the large intestine, i.e., the cecum, colon, rectum, and anal canal. Bacteria can be found throughout the intestine. As used herein, mouth encompasses the tissues in the mouth, e.g., any soft tissue in the mouth of a mammal, the pharynx, etc.

In some embodiments, different portions of the intestine may be associated with disease. In CD, for example, the bacteriophage may be formulated to target bacteria present in the small intestine, colon, ileum, ileocecal junction, esophagus, mouth, and/or anus. In UC, for example, the bacteriophage may be formulated to target bacteria present in the colon, rectum, anus, and large intestine of a mammal.

The articles "a" and "an," as used herein, should be understood to mean "at least one," unless clearly indicated to the contrary.

The phrase "and/or," when used between elements in a list, is intended to mean either (1) that only a single listed element is present, or (2) that more than one element of the list is present. For example, "A, B, and/or C" indicates that the selection may be A alone; B alone; C alone; A and B; A and C; B and C; or A, B, and C. The phrase "and/or" may be used interchangeably with "at least one of" or "one or more of" the elements in a list.

All ranges include end points. All references cited are incorporated for any purpose (specification controls where there are inconsistencies). Singular form includes plural.

### Bacteriophage

The bacteriophage described herein is capable of lysing one or more *Klebsiella* bacterial strains that are associated with IBD, e.g., ulcerative colitis, Crohn's disease. In some embodiments, the bacteriophage is capable of lysing one or more *Klebsiella pneumoniae* bacterial strains that are associated with IBD. In some embodiments, the bacteriophage is capable of lysing one or more *Klebsiella aeromobilis* bacterial strains that are associated with IBD. In some embodiments, the bacteriophage is capable of modulating IBD by lysing the one or more *Klebsiella* bacteria in a mammal. In some embodiments, the bacteriophage is capable of modulating IBD by lysing the one or more *Klebsiella* bacteria in the intestine of a mammal. In some embodiments, the bacteriophage is capable of modulating IBD by lysing the one or more *Klebsiella* bacteria in the mouth of a mammal.

In some embodiments, the bacteriophage is capable of modulating IBD associated with proton pump inhibitor (PPI) therapy (Juillerat et al., 2012) by lysing the one or more *Klebsiella* bacteria. In some embodiments, the bacteriophage is capable of modulating IBD associated with primary sclerosing cholangitis (Hirschfield et al., 2013; Palmela et al., 2017) by lysing the one or more *Klebsiella* bacteria.

In some embodiments, the bacteriophage is selected from KP2W-P0101, KP2W-P0102, KP2W-P0103, KP2W-P0104, KP2W-P0105, KP2W-P0106, KP2W-P0107, KP2W- P0108, and KP2W-P0109. In some embodiments, the bacteriophage is additionally selected from a bacteriophage that comprises at least about 83%, at least about 85%, at least about 88%, at least about 90%, at least about 95%, at least about 97%, or at least about 99% homology to the sequence of KP2W-P0101 as measured by BLASTN. The bacteriophages identified in this paragraph are referred to as "Group 1" bacteriophage and are capable of infecting the bacterial strains described herein, *see, e.g.,* Fig. 3 and Fig. 5.

In some embodiments, the bacteriophage is selected from KP2M-P0201, KP2W-P0202, KP2M-P0203, KP2M-P0204, KP2M-P0205, KP2M-P0206, KP2M-P0207, KP2M-P0208, KP2M-P0209, KP2M-P0210, KP2M-P0211, KP2M-P0212. In some embodiments, the bacteriophage is additionally selected from a bacteriophage that comprises at least about 85%, at least about 86%, at least about 88%, at least about 90%, at least about 95%, at least about 97%, or at least about 99% homology to the sequence of KP2M-P0201 as measured by BLASTN. The bacteriophages identified in this paragraph are referred to as "Group 2" bacteriophage and are capable of infecting the bacterial strains described herein, *see, e.g.,* Fig. 3 and Fig. 5.

In some embodiments, the bacteriophage is selected from KP2W-M0102, KP2W-M0103, KP2W-M0104, KP2M-M0105, KP2W-M0106, KP2W-M0107, KP2M-M0108, KP2M-M0109, KP2M-M0110, KP2M-M0111, KP2M-M0112. In some embodiments, the bacteriophage is additionally selected from a bacteriophage that comprises at least about 86%, at least about 88%, at least about 90%, at least about 95%, at least about 97%, or at least about 99% homology to the sequence of KP2W-M0102 as measured by BLASTN. The bacteriophages identified in this paragraph are referred to as "Group 3" bacteriophage and are capable of infecting the bacterial strains described herein, *see, e.g.,* Fig. 3 and Fig. 5.

In some embodiments, the bacteriophage is selected from KP3W-P0101, KP3W-P0102, KP3W-P0103, KP3W-P0104, KP3W-P0105, KP3W-P0106, KP3W-P0107, KP3W-P0108, KP3W-P0109, KP3W-P0110, KP3W-P0111, KP3W-P0112, KP3W-P0113, KP3W-P0114, KP3W-P0201, KP3W-P0202, and KP3W-P0203. In some embodiments, the bacteriophage comprises at least about 78%, at least about 80%, at least about 82%, at least about 85%, at least about 88%, at least about 90%, at least about 95%, at least about 97%, or at least about 99% homology to the sequence of KP3W-P0101 as measured by BLASTN. In some embodiments, the bacteriophage comprises at least about 95%, at least about 96%, at least about 97%, or at least about 99% homology to the sequence of KP3W-P0201 as measured by BLASTN. The bacteriophages identified in this paragraph are referred to as "Group 4" bacteriophage and are capable of infecting the bacterial strains described herein. *See, e.g.,* Fig. 3 and Fig. 6.

In some embodiments, the bacteriophage is selected from KP3W-S0101, and KP3W-S0102. In some embodiments, the bacteriophage is additionally selected from a bacteriophage that comprises at least about 88%, at least about 90%, at least about 95%, at least about 97%, or at least about 99% homology to the sequence of KP3W-P0201 as measured by BLASTN. The bacteriophages identified in this paragraph are referred to as "Group 5" bacteriophage and are capable of infecting the bacterial strains described herein. *See, e.g.,* Fig. 3 and Fig. 6.

In some embodiments, the bacteriophage is capable of infecting KP2, but not capable of infecting any of ATCC BAA-2552 (KP1), KP3, ATCC 23356 (KP4), ATCC 13882 (KP5), ATCC BAA-1705 (KP6), ATCC 700603 (KP7), or ATCC 700721(KP8). *See, e.g.,* Fig. 3 and Fig. 5.

In some embodiments, the bacteriophage is capable of infecting KP3, but not capable of infecting any of ATCC BAA-2552 (KP1), KP2, ATCC 23356 (KP4), ATCC 13882 (KP5), ATCC BAA-1705 (KP6), ATCC 700603 (KP7), or ATCC 700721(KP8). *See, e.g.,* Fig. 3 and Fig. 5.

In some embodiments, the bacteriophage is capable of infecting KP2 and KP4, but not capable of infecting any of ATCC BAA-2552 (KP1), KP3, ATCC 13882 (KP5), ATCC BAA-1705 (KP6), ATCC 700603 (KP7), or ATCC 700721(KP8). *See, e.g.,* Fig. 3 and Fig. 5.

In some embodiments, during phage infection, mutant bacterial strains arise that are resistant to the phage. In some embodiments, it is beneficial for a phage to target the mutant bacteria. To produce said phage, bacteria may be incubated with one or more phage(s) disclosed herein to generate mutant bacterial strains. Environmental and clinical samples may then be screened for phage that are capable of infecting the mutant bacterial strains in order to identify the new phage. Then the titer of the phage(s) is selected to allow for efficient attachment, penetration, amplification and release of new phage(s) from the bacterial cells. Exemplary mutant KP2 strains are described herein. *See, e.g.,* Table 1 (contigs). In some embodiments, the mutant bacterial strain mirrors mutations that are likely to occur *in vivo* when a subject is treated with the phage disclosed herein. In some embodiments, a bacteriophage may be generated that is capable of infecting and lysing the mutant bacterial strain. Thus, in some embodiments, the bacteriophages provided herein are capable of treating unmodified KP2 and/or KP3 bacteria, as well as resistant KP2 and/or KP3 mutants.

In some embodiments, the bacteriophage is capable of infecting KP2 and at least one bacteria, at least two bacteria, at least three bacteria, at least four bacteria, or at least five bacteria selected from MKP2_2161_1, MKP2_2161_C, MKP2_251_B, MKP2_251_C, MKP2_251_D, and MKP2_coc_1. *See, e.g.,* Fig. 5.

In some embodiments, the bacteriophage is capable of infecting KP2, KP4, and at least one bacteria, at least two bacteria, at least three bacteria, at least four bacteria, or at least five bacteria selected from MKP2_2161_1, MKP2_2161_C, MKP2_251_B, MKP2_251_C, MKP2_251_D, and MKP2_coc_1. *See, e.g.,* Fig. 5.

In some embodiments, the bacteriophage is capable of infecting at least one bacteria selected from MKP2_2161_1, MKP2_2161_C, MKP2_251_B, MKP2_251_C, MKP2_251_D, and MKP2_coc_1. *See, e.g.,* Fig. 5.

As used herein, a lytic bacteriophage "cocktail" refers to a composition comprising at least two different isolates of lytic bacteriophage as described herein. As used herein, "a" or "one" bacteriophage refers to an isolate or type of bacteriophage and is not necessarily intended to refer to a single bacteriophage particle.

In some embodiments, the cocktail comprises at least two different KP2 bacteriophages described herein and is capable of infecting wild-type KP2 bacteria. In some embodiments, the cocktail comprises at least two different KP2 bacteriophages described herein and is capable of infecting mutant KP2 bacteria. In some embodiments, the cocktail comprises at least two different KP2 bacteriophages described herein and is capable of infecting wild-type KP2 bacteria and mutant KP2 bacteria.

In some embodiments, the cocktail comprises at least two different KP3 bacteriophages described herein and is capable of infecting wild-type KP3 bacteria. In some embodiments, the cocktail comprises at least two different KP3 bacteriophages described herein and is capable of infecting mutant KP3 bacteria. In some embodiments, the cocktail comprises at least two different KP3 bacteriophages described herein and is capable of infecting wild-type KP3 bacteria and mutant KP3 bacteria.

In some embodiments, the cocktail comprises at least two different types of KP2 bacteriophages and no KP3 bacteriophages. In some embodiments, the cocktail comprises at least two different types of KP3 bacteriophages and no KP2 bacteriophages. In some embodiments, the cocktail comprises at least one type of KP2 bacteriophage and at least one type of KP3 bacteriophage. In some embodiments, the cocktail comprises only one type of KP2 bacteriophage and only one type of KP3 bacteriophage. In some embodiments, a cocktail comprising at least two phages widens the host range as compared to a single phage. In some embodiments, a cocktail comprising at least two phages reduces the appearance of phage-resistant bacteria as compared to a single phage.

In some embodiments, the cocktail comprises at least one KP2 bacteriophage and at least one KP3 bacteriophage described herein and is capable of infecting wild-type KP2 bacteria and wild-type KP3 bacteria. In some embodiments, the cocktail comprises at least one KP2 bacteriophage and at least one KP3 bacteriophage described herein and is capable of infecting wild-type KP2 bacteria, mutant phage-resistant KP2, and/or wild-type KP3 bacteria. In some embodiments, the cocktail comprises at least one KP2 bacteriophage and at least one KP3 bacteriophage described herein and is capable of infecting wild-type KP2 bacteria, mutant phage-resistant KP2, wild-type KP3 bacteria, and/or mutant phage-resistant KP3.

In some embodiments, the cocktail comprises KP2W-P0105 + KP2M-P0203; KP2M-P0203 + KP2W-M0104; KP2W-P0105 + KP2W-M0104; KP2W-P0102 + KP2M-P0209; KP2M-P0209 + KP2W-M0107; KP2W-P0102 + KP2W-M0107; KP2W-P0109 + KP2M-P0211; KP2M-P0211 + KP2M-M0112; or KP2W-P0109 + KP2M-M0112. In some embodiments, the cocktail comprises at least one bacteriophage selected from KP2W-P0105, KP2W-P0102, and KP2W-P0109; at least one bacteriophage selected from KP2M-P0203, KP2M-P0209, and KP2M-P0211; and at least one bacteriophage selected from KP2W-M0104, KP2W-M0107, and KP2M-M0112. In some embodiments, the cocktail comprises KP2W-P0105 + KP2M-P0203 + KP2W-M0104; KP2W-P0102 + KP2M-P0209 + KP2W-M0107; KP2W-P0109 + KP2M-P0211 + KP2M-M0112; KP2W-P0105 + KP2M-P0209 + KP2M-M0112 KP2W-P0102 + KP2M-P0211 + KP2W-M0104; or KP2W-P0109 + KP2M-P0203 + KP2W-M0107.

In some embodiments, the bacteriophage or cocktail does not significantly affect the subject's microbiota. In some embodiments, the bacteriophage or cocktail does not significantly affect the subject's commensal bacteria. In some embodiments, the bacteriophage or cocktail does not significantly affect the subject's probiotic bacteria. In some embodiments, a bacteriophage or cocktail is not naturally capable of affecting the microbiota, commensal bacteria, or probiotic bacteria, because the bacteriophage's natural host range is limited, e.g., to KP2 or KP3.

In some embodiments, a bacteriophage that is specific for a bacterium is capable of recognizing specific receptors and/or attachment sites expressed exclusively by said bacterium. In some embodiments, the KP2 bacteriophage is "specific" for KP2 bacteria. For example, the KP2 bacteriophage is capable of infecting and/or productively creating more virions in the KP2 bacteria in an *in vitro* or *in vivo* assay, e.g., in a plaque assay, an OD assay, a phage-adsorption assay, or any other assay showing that the bacteriophage can enter, reproduce, and exit the bacteria. In some embodiments, the KP3 bacteriophage is "specific" for KP3 bacteria. For example, the KP3 bacteriophage is capable of infecting and/or productively creating more virions in the KP3 bacteria in an *in vitro* or *in vivo* assay, e.g., in a plaque assay, an OD assay, a phage-adsorption assay, or any other assay showing that the bacteriophage can enter, reproduce, and exit the bacteria.

### Bacterial lysis

In some embodiments, the bacteriophage provided herein is capable of lysing deleterious *Klebsiella* bacteria that induce immune and/or inflammatory response(s). In some embodiments, the bacteriophage provided herein is capable of lysing deleterious *Klebsiella* bacteria that disrupt the balance of immune homeostasis in the intestine, e.g., by enhancing the pro-inflammatory activity, ultimately resulting in chronic intestinal inflammation. In some embodiments, the bacteriophage provided herein is capable of lysing deleterious *Klebsiella* bacteria that induce a T helper 1 (TH1) cell response. In some embodiments, the *Klebsiella* bacteria to be lysed by the bacteriophage provided herein are in the lumen. In some embodiments, the *Klebsiella* bacteria to be lysed by the bacteriophage provided herein are associated with the epithelium. In some embodiments, the *Klebsiella* bacteria to be lysed by the bacteriophage provided herein are in between the mucosal and submucosal layer in the ileum, e.g., in Crohn's disease (Chiodini et al., 2015).

In some embodiments, the bacteriophage provided herein is capable of lysing deleterious KP2 and/or KP3 bacteria that are associated with IBD, e.g., ulcerative colitis or Crohn's disease, and may be administered to ameliorate the disease or at least one symptom thereof. In some embodiments, there is a higher incidence of KP2 bacteria associated with Crohn's disease as compared to ulcerative colitis. However, in embodiments where a subject has ulcerative colitis associated with KP2 bacterial presence, the bacteriophage provided herein may be administered to lyse those bacteria and ameliorate the ulcerative colitis or at least one symptom thereof.

Drugs that inhibit gastric acid secretion, e.g., proton pump inhibitors, and primary sclerosing cholangitis (PSC) may alter the course of IBD. In some embodiments, the bacteriophage provided herein is capable of lysing deleterious KP2 and/or KP3 bacteria that are associated proton pump inhibitor therapy or PSC, and may be administered to ameliorate at least one symptom of IBD associated with proton pump inhibitor therapy or PSC.

### Pharmaceutical compositions

Pharmaceutical compositions comprising the bacteriophage described herein may be used to modulate IBD. Pharmaceutical compositions comprising one or more bacteriophage, alone or in combination with prophylactic agents, therapeutic agents, and/or and pharmaceutically acceptable carriers are provided. In certain embodiments, the pharmaceutical composition comprises one bacteriophage described herein. In alternate embodiments, the pharmaceutical composition comprises two or more bacteriophages described herein.

The pharmaceutical compositions described herein may be formulated in a conventional manner using one or more physiologically acceptable carriers comprising excipients and auxiliaries, which facilitate processing of the active ingredients into compositions for pharmaceutical use. Methods of formulating pharmaceutical compositions are known in the art (see, e.g., "Remington's Pharmaceutical Sciences," Mack Publishing Co., Easton, PA). In some embodiments, the pharmaceutical compositions are subjected to tableting, lyophilizing, direct compression, conventional mixing, dissolving, granulating, levigating, emulsifying, encapsulating, entrapping, or spray drying to form tablets, granulates, nanoparticles, nanocapsules, microcapsules, microtablets, pellets, or powders, which may be enterically coated or uncoated. Appropriate formulation depends on the route of administration.

The bacteriophage described herein may be formulated into pharmaceutical compositions in any suitable dosage form (e.g., liquids, capsules, sachet, hard capsules, soft capsules, tablets, enteric coated tablets, suspension powders, granules, or matrix sustained release formations for oral administration) and for any suitable type of administration (e.g., oral, topical, injectable, immediate-release, pulsatile-release, delayed-release, or sustained release). In some embodiments the bacteriophage are formulated for administration as an oral rinse, a lozenge, a strip, or gum. The composition may be administered once or more daily, weekly, or monthly.

The bacteriophage may be formulated into pharmaceutical compositions comprising one or more pharmaceutically acceptable carriers, thickeners, diluents, buffers, buffering agents, surface active agents, neutral or cationic lipids, lipid complexes, liposomes, penetration enhancers, carrier compounds, and other pharmaceutically acceptable carriers or agents. For example, the pharmaceutical composition may include, but is not limited to, the addition of calcium bicarbonate, sodium bicarbonate, calcium phosphate, various sugars and types of starch, cellulose derivatives, gelatin, vegetable oils, polyethylene glycols, and surfactants, including, for example, polysorbate 20. In some embodiments, the bacteriophage may be formulated in a solution of sodium bicarbonate, e.g., 1 molar solution of sodium bicarbonate (to buffer an acidic cellular environment, such as the stomach, for example). The bacteriophage may be administered and formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The bacteriophage may be administered orally and formulated as tablets, pills, dragees, capsules, liquids, gels, syrups, slurries, suspensions, etc. Pharmacological compositions for oral use can be made using a solid excipient, optionally grinding the resulting mixture, and processing the mixture of granules, after adding suitable auxiliaries if desired, to obtain tablets or dragee cores. Suitable excipients include, but are not limited to, fillers such as sugars, including lactose, sucrose, mannitol, or sorbitol; cellulose compositions such as maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP) or polyethylene glycol (PEG). Disintegrating agents may also be added, such as cross-linked polyvinylpyrrolidone, agar, alginic acid or a salt thereof such as sodium alginate.

In some embodiments, the bacteriophage is formulated for delivery to a mammalian mouth. In some embodiments, the pharmaceutical composition comprises the bacteriophage and a pharmaceutically acceptable excipient, wherein the bacteriophage and the pharmaceutically acceptable excipient do not occur together in nature. In some embodiments, the pharmaceutical composition comprises the bacteriophage and a pharmaceutically acceptable excipient, wherein the pharmaceutically acceptable excipient is a non-naturally occurring excipient.

In some embodiments, the bacteriophage is formulated for delivery to a mammalian intestine. In some embodiments, the pharmaceutical composition comprises the bacteriophage and a pharmaceutically acceptable excipient, wherein the bacteriophage and the pharmaceutically acceptable excipient do not occur together in nature. In some embodiments, the pharmaceutical composition comprises the bacteriophage and a pharmaceutically acceptable excipient, wherein the pharmaceutically acceptable excipient is a non-naturally occurring excipient.

Tablets or capsules can be prepared by conventional means with pharmaceutically acceptable excipients such as binding agents (e.g., pregelatinized maize starch, polyvinylpyrrolidone, hydroxypropyl methylcellulose, carboxymethylcellulose, polyethylene glycol, sucrose, glucose, sorbitol, starch, gum, kaolin, and tragacanth); fillers (e.g., lactose, microcrystalline cellulose, or calcium hydrogen phosphate); lubricants (e.g., calcium, aluminum, zinc, stearic acid, polyethylene glycol, sodium lauryl sulfate, starch, sodium benzoate, L-leucine, magnesium stearate, talc, or silica); disintegrants (e.g., starch, potato starch, sodium starch glycolate, sugars, cellulose derivatives, silica powders); or wetting agents (e.g., sodium lauryl sulphate). The tablets may be coated by methods well known in the art. A coating shell may be present, and common membranes include, but are not limited to, polylactide, polyglycolic acid, polyanhydride, other biodegradable polymers, alginate-polylysine-alginate (APA), alginate-polymethylene-co-guanidine-alginate (A-PMCG-A), hydroxymethylacrylate-methyl methacrylate (HEMA-MMA), multilayered HEMA-MMA-MAA, polyacrylonitrilevinylchloride (PAN-PVC), acrylonitrile/sodium methallylsulfonate (AN-69), polyethylene glycol/poly pentamethylcyclopentasiloxane/polydimethylsiloxane (PEG/PD5/PDMS), poly N,N-dimethyl acrylamide (PDMAAm), siliceous encapsulates, cellulose sulphate/sodium alginate/polymethylene-co-guanidine (CS/A/PMCG), cellulose acetate phthalate, calcium alginate, k-carrageenan-locust bean gum gel beads, gellan-xanthan beads, poly(lactide-co-glycolides), carrageenan, starch poly-anhydrides, starch polymethacrylates, polyamino acids, and enteric coating polymers.

In some embodiments, the bacteriophage is enterically coated for release into the gut or a particular region of the gut. The typical pH profile from the stomach to the colon is about 1-4 (stomach), 5.5-6 (duodenum), 7.3-8.0 (ileum), and 5.5-6.5 (colon). In some diseases, the pH profile may be modified. In some embodiments, the coating is degraded in specific pH environments in order to specify the site of release.

Liquid preparations for oral administration may take the form of solutions, syrups, suspensions, or a dry product for constitution with water or other suitable vehicle before use. Such liquid preparations may be prepared by conventional means with pharmaceutically acceptable agents such as suspending agents (e.g., sorbitol syrup, cellulose derivatives, or hydrogenated edible fats); emulsifying agents (e.g., lecithin or acacia); nonaqueous vehicles (e.g., almond oil, oily esters, ethyl alcohol, or fractionated vegetable oils); and preservatives (e.g., methyl or propyl-p-hydroxybenzoates or sorbic acid). The preparations may also contain buffer salts, flavoring, coloring, and sweetening agents as appropriate. Preparations for oral administration may be suitably formulated for slow release, controlled release, or sustained release of the bacteriophage.

In certain embodiments, the bacteriophage may be orally administered, for example, with an inert diluent or an assimilable edible carrier. The compound may also be enclosed in a hard or soft shell gelatin capsule, compressed into tablets, or incorporated directly into the subject's diet. For oral therapeutic administration, the compounds may be incorporated with excipients and used in the form of ingestible tablets, buccal tablets, troches, capsules, elixirs, suspensions, syrups, wafers, and the like. To administer a compound by other than parenteral administration, it may be necessary to coat the compound with, or co-administer the compound with, a material to prevent its inactivation. In some embodiments, the bacteriophage is orally administered for delivery to the mouth and is capable of lysing bacteria present in the mouth. In some embodiments, the bacteriophage is orally administered for delivery to the intestine and is capable of lysing bacteria present in the intestine.

In some embodiments, the composition is formulated for intraintestinal administration, intrajejunal administration, intraduodenal administration, intraileal administration, gastric shunt administration, or intracolic administration, via nanoparticles, nanocapsules, microcapsules, or microtablets, which are enterically coated or uncoated. The pharmaceutical compositions may also be formulated in rectal compositions such as suppositories or retention enemas, using, e.g., conventional suppository bases such as cocoa butter or other glycerides. The compositions may be suspensions, solutions, or emulsions in oily or aqueous vehicles, and may contain suspending, stabilizing and/or dispersing agents.

In some embodiments, the pharmaceutically acceptable composition is in single dosage form. Single dosage forms may be in a liquid or a solid form. Single dosage forms may be administered directly to a patient without modification or may be diluted or reconstituted prior to administration. In certain embodiments, a single dosage form may be administered in bolus form, e.g., single injection, single oral dose, including an oral dose that comprises multiple tablets, capsule, pills, etc. In alternate embodiments, a single dosage form may be administered over a period of time, e.g., by infusion. Single dosage forms of the pharmaceutical composition may be prepared by portioning the pharmaceutical composition into smaller aliquots, single dose containers, single dose liquid forms, or single dose solid forms, such as tablets, granulates, nanoparticles, nanocapsules, microcapsules, microtablets, pellets, or powders, which may be enterically coated or uncoated. A single dose in a solid form may be reconstituted by adding liquid, typically sterile water or saline solution, prior to administration to a patient.

In other embodiments, the composition can be delivered in a controlled release or sustained release system. In one embodiment, a pump may be used to achieve controlled or sustained release. In another embodiment, polymeric materials can be used to achieve controlled or sustained release of the therapies of the present disclosure (see, e.g., U.S. Patent No. 5,989,463). Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N- vinyl pyrrolidone), poly(vinyl alcohol), polyacrylamide, poly(ethylene glycol), polylactides (PLA), poly(lactide-co-glycolides) (PLGA), and polyorthoesters. The polymer used in a sustained release formulation may be inert, free of leachable impurities, stable on storage, sterile, and biodegradable. In some embodiments, a controlled or sustained release system can be placed in proximity of the prophylactic or therapeutic target, thus requiring only a fraction of the systemic dose. Any suitable technique known to one of skill in the art may be used.

Dosage regimens may be adjusted to provide a therapeutic response. Dosing can depend on several factors, including severity and responsiveness of the disease, route of administration, time course of treatment (days to months to years), and time to amelioration of the disease. For example, a single bolus may be administered at one time, several divided doses may be administered over a predetermined period of time, or the dose may be reduced or increased as indicated by the therapeutic situation.

In some embodiments, the ingredients are supplied either separately or mixed together in unit dosage form. The pharmaceutical compositions may be packaged in a hermetically sealed container such as an ampoule or sachet indicating the quantity of the agent. In one embodiment, one or more of the pharmaceutical compositions is supplied as a dry sterilized lyophilized powder or water-free concentrate in a hermetically sealed container and can be reconstituted (e.g., with water or saline) to the appropriate concentration for administration to a subject. In an embodiment, one or more of the prophylactic or therapeutic agents or pharmaceutical compositions is supplied as a dry sterile lyophilized powder in a hermetically sealed container and reconstituted prior to administration. Cryoprotectants can be included for a lyophilized dosage form, principally 0-10% sucrose (optimally 0.5-1.0%). Other suitable cryoprotectants include trehalose and lactose. Other suitable bulking agents include glycine and arginine, either of which can be included at a concentration of 0-0.05%, and polysorbate-80 (optimally included at a concentration of 0.005-0.01%). Additional surfactants include but are not limited to polysorbate 20 and BRIJ surfactants. The pharmaceutical composition may be prepared as an injectable solution and can further comprise an agent useful as an adjuvant, such as those used to increase absorption or dispersion, e.g., hyaluronidase.

### Method of treatment

Methods of treating IBD in a mammal using a bacteriophage, a bacteriophage cocktail, and/or a pharmaceutical composition comprising a bacteriophage or a bacteriophage cocktail are provided. In some embodiments, a method of treating IBD comprises administering to a subject the KP2 and/or KP3 bacteriophage described herein. In some embodiments, a method of treating Crohn's disease comprises administering to a subject the KP2 and/or KP3 bacteriophage described herein. In some embodiments, a method of treating ulcerative colitis comprises administering to a subject the KP2 and/or KP3 bacteriophage described herein. In some embodiments, a method of treating IBD associated with primary sclerosing cholangitis comprises administering to a subject the KP2 and/or KP3 bacteriophage described herein. In some embodiments, IBD may be associated with proton pump inhibitor therapy, and a subject may be administered the KP2 and/or KP3 bacteriophage described herein before, during, or after proton pump inhibitor therapy.

In some embodiments, a method of treating IBD comprises administering to a mammal determined to be infected with *Klebsiella* bacteria the bacteriophage, the bacteriophage cocktail, and/or the pharmaceutical composition comprising a bacteriophage or a bacteriophage cocktail described herein. In some embodiments, a method of treating IBD comprises administering to a mammal determined to be infected with *Klebsiella pneumoniae* bacteria the bacteriophage, the bacteriophage cocktail, and/or the pharmaceutical composition comprising a bacteriophage or a bacteriophage cocktail described herein. In some embodiments, a method of treating IBD comprises administering to a mammal determined to be infected with *Klebsiella pneumoniae* KP2 bacteria the bacteriophage, the bacteriophage cocktail, and/or the pharmaceutical composition comprising a bacteriophage or a bacteriophage cocktail described herein. In some embodiments, a method of treating IBD comprises administering to a mammal determined to be infected with *Klebsiella aeromobilis* bacteria the bacteriophage, the bacteriophage cocktail, and/or the pharmaceutical composition comprising a bacteriophage or a bacteriophage cocktail described herein. In some embodiments, a method of treating IBD comprises administering to a mammal determined to be infected with *Klebsiella aeromobilis* KP3 bacteria the bacteriophage, the bacteriophage cocktail, and/or the pharmaceutical composition comprising a bacteriophage or a bacteriophage cocktail described herein.

In some embodiments, the bacteriophage and the method of treatment is used prophylactically. For example, the method comprises administering to a mammal determined to be susceptible to IBD the bacteriophage, the bacteriophage cocktail, and/or the pharmaceutical composition comprising a bacteriophage or a bacteriophage cocktail described herein.

In some embodiments, susceptibility to IBD is measured by the genetic susceptibility to acquire IBD, as determined by the presence of mutations in the genes or loci associated with the immune function in the intestine including loci and genes involved in inflammation and immunoregulation, tissue remodeling, tumorigenesis and apoptosis (for example, NOD2, HLA-27, ATG16L1, interleukins, CCL and CCR genes, SOD genes, MMPs, JAK, STAT, TIMP genes and many others as described in Suzuki et al., 2012) and genes associated with autophagy (Jostins et al., 2012); presence of other immune conditions (sibling diseases) such as autoimmune disorder, Behcet's disease, graft versus host disease, systemic lupus erythematosus, dermatomyositis, polymyositis, auto-immune chronic active hepatitis, pemphigus vulgaris, polyarteritis nodosa, auto-immune hemolytic anaemia, idiopathic thrombocytopenic purpura, myasthenia gravis, lupus nephritis, platelet transfusion refractoriness type 1 diabetes, ankylosing spondylitis, multiple sclerosis, psoriasis, psoriatic arthritis, asthma, and rheumatoid arthritis (Suzuki et al., 2012); acute gastroenteritis (Raposo et al., 2017); dietary constituents that have been shown to affect the immune response and the inflammatory status, through the modulation of the microbiota, for example high carbohydrate and animal fat diet, high intake of red meat and processed meat, protein, alcoholic beverages, sulfur, and sulfate, processed food (Raposo et al., 2017); lifestyle habits (Western hygiene standards and improved sanitary conditions); medications (antibiotics and steroids) especially when used in childhood leading to the alterations in the microbiome composition (Raposo et al., 2017); epigenetic mechanisms dependent on the nutrients e.g. folates and selenium (DNA methylation (Raposo et al., 2017); pollutants, sedentary lifestyle and tobacco-smoking (Raposo et al., 2017); geography (Hold at al., 2014); history of the IBD in the family (Baumgart and Sandborn, 2012; Neurath, 2014).

In some embodiments, the bacteriophage is administered more than once to achieve a desired therapeutic effect. For example, when a host bacterium is destroyed, the bacteriophage that infected said bacterium can no longer multiply because its host has been eradicated and may be eliminated from the digestive tract, and the bacteriophage may need to be re-administered, e.g., at least twice daily, at least daily, at least weekly, or at least monthly.

In some embodiments, the phage described herein may be administered in combination with one or more known and suitable medicaments for IBD, including antiinflammatory drugs, dietary therapy, probiotics, etc.

### Methods of determining a subject to be treated with phage

Methods of selecting subjects that are responsive to treatment are provided herein. In some embodiments, the method of selecting a subject that is responsive to treatment by the methods set forth herein comprises (1) obtaining a biological sample, e.g., from the intestine or mouth of the subject, (2) culturing the bacteria obtained from the biological sample, (3) inoculating the cultured bacteria with a bacteriophage described herein, and (4) determining the amount of cultured bacteria that are lysed by the bacteriophage. In some embodiments, the lysis is determined by a liquid media assay and/or a solid media assay as described herein. In some embodiments, the amount of lysis using the bacteria cultured from the intestinal sample is compared to a control, e.g., a sample obtained from a different, uninfected subject or a sample from the same subject prior to infection. In some embodiments, the phage described herein is suitable for treating KP infection if any lysis is mediated by the phage *in vitro* and/or *in vivo.* In some embodiments, a subject is determined to be infected with *Klebsiella* if a detectable portion of bacteria cultured from the subject are lysed by the bacteriophage. In some embodiments, a subject is determined to be infected with *Klebsiella pneumoniae* KP2 if a detectable portion of the cultured bacteria are lysed by a KP2 bacteriophage, see, e.g., Table 5. In some embodiments, a subject is determined to be infected with *Klebsiella aeromobilis* KP3 if a detectable portion of the cultured bacteria are lysed by a KP3 bacteriophage, e.g., KP3W-P0101, KP3W-P0102, KP3W-P0103, KP3W-P0104, KP3W-P0105, KP3W-P0106, KP3W-P0107, KP3W-P0108, KP3W-P0109, KP3W-P0110, KP3W-P0111, KP3W-P0112, KP3W-P0113, KP3W-P0114, KP3W-P0201, KP3W-P0202, KP3W-P0203, KP3W-S0101, KP3W-S0101, or variants or mutants thereof.

In some embodiments, the biological sample is an oral sample or expectorate, a biopsy, or stool. In some embodiments, determining the amount of cultured bacteria that are lysed by the bacteriophage comprises performing a KP2-specific qPCR of the bacterial sample, a KP3-specific qPCR of the bacterial sample, and/or a direct test for phage sensitivity using the plaque assay or liquid OD method described herein. In some embodiments, a positive sample or subject to be treated is determined by the presence of plaque in a plaque assay. In some embodiments, a positive sample or subject to be treated is determined by a reduction in OD in a liquid OD assay. In some embodiments, serotype-based screening is performed using an assay, e.g., an immunoassay, an ELISA, to determine the presence of antibodies against KP2, KP3, and/or their respective capsular types. In some embodiments, the phage is labeled with a detectable marker, e.g., a luminescent marker, and the infection of the bacteria is determined by detecting an increase in the marker, e.g., in a luminescence assay.

### References

1. Abedon, S. T., & Yin, J. (2009). Bacteriophage plaques: theory and analysis. Methods in molecular biology (Clifton, N.J.), 501, 161-74. doi:10.1007/978-1-60327-164-6_17
2. Altschul et al., 1997. Gapped BLAST and PSI-BLAST: a new generation of protein database search programs, Nucleic Acids Res. 25:3389-3402.
3. Baym et al. (2015. Inexpensive multiplexed library preparation for megabase-sized genomes. PLoS ONE, 10(5), 1-15)
4. Y. Chen et al., Dysbiosis of small intestinal microbiota in liver cirrhosis and its association with etiology. Scientific reports 6, 34055 (Sep 30, 2016).
5. Cole et al. 2014. Ribosomal Database Project: data and tools for high throughput rRNA analysis Nucl. Acids Res. 42:D633-D642
6. A. Davin-Regli, J. M. Pages, Enterobacter aerogenes and Enterobacter cloacae; versatile bacterial pathogens confronting antibiotic treatment. Frontiers in microbiology 6, 392 (2015).
7. Diene et al. The rhizome of the multidrug-resistant Enterobacter aerogenes genome reveals how new "killer bugs" are created because of a sympatric lifestyle. Mol Biol Evol. 2013 Feb;30(2):369-83. PubMed PMID: 23071100.
8. D. Gevers et al., The treatment-naive microbiome in new-onset Crohn's disease. Cell host & microbe 15, 382 (Mar 12, 2014).
9. J. H. Grose et al., Understanding the enormous diversity of bacteriophages: the tailed phages that infect the bacterial family Enterobacteriaceae. Virology 0,421 (November 2014).
10. K. E. Holt et al., Genomic analysis of diversity, population structure, virulence, and antimicrobial resistance in Klebsiella pneumoniae, an urgent threat to public health. Proceedings of the National Academy of Sciences of the United States of America 112, E3574 (Jul 07, 2015).
11. Hyman, P., & Abedon, S. T. (2010). Bacteriophage host range and bacterial resistance. Advances in applied microbiology (1st ed., Vol. 70, pp. 217-48). Elsevier Inc. doi:10.1016/S0065-2164(10)70007-1
12. Kazutaka Katoh, Daron M. Standley; MAFFT Multiple Sequence Alignment Software Version 7: Improvements in Performance and Usability. Mol Biol Evol 2013; 30 (4): 772-780
13. Kutter, E. (2009). Phage host range and efficiency of plating. Methods in molecular biology (Clifton, N.J.), 501, 141-9. doi:10.1007/978-l-60327-164-6_14
14. C. A. Lozupone et al., Alterations in the gut microbiota associated with HIV-1 infection. Cell host & microbe 14, 329 (Sep 11, 2013).
15. S. Mondot et al., Highlighting new phylogenetic specificities of Crohn's disease microbiota. Inflammatory bowel diseases 17, 185 (Jan, 2011).
16. E. G. Pamer, Resurrecting the intestinal microbiota to combat antibiotic-resistant pathogens. Science 352, 535 (Apr 29, 2016).
17. N. Qin et al., Alterations of the human gut microbiome in liver cirrhosis. Nature 513, 59 (Sep 04, 2014).
18. H. S. Said et al., Dysbiosis of salivary microbiota in inflammatory bowel disease and its association with oral immunological biomarkers. DNA research : an international journal for rapid publication of reports on genes and genomes 21, 15 (Feb, 2014).
19. C. L. Sears, W. S. Garrett, Microbes, microbiota, and colon cancer. Cell host & microbe 15, 317 (Mar 12, 2014).
20. E. S. Snitkin et al., Tracking a hospital outbreak of carbapenem-resistant Klebsiella pneumoniae with whole-genome sequencing. Science translational medicine 4, 148ra116 (Aug 22, 2012).
21. Y. Taur, E. G. Pamer, The intestinal microbiota and susceptibility to infection in immunocompromised patients. Current opinion in infectious diseases 26, 332 (Aug, 2013).
22. Tindall et al. Enterobacter aerogenes Hormaeche and Edwards 1960 (Approved Lists 1980) and Klebsiella mobilis Bascomb et al. 1971 (Approved Lists 1980) share the same nomenclatural type (ATCC 13048) on the Approved Lists and are homotypic synonyms, with consequences for the name Klebsiella mobilis Bascomb et al. 1971 (Approved Lists 1980). Int J Syst Evol Microbiol. 2017 Feb;67(2):502-504. PubMed PMID: 27902205.
23. I. Vujkovic-Cvijin et al., Dysbiosis of the gut microbiota is associated with HIV disease progression and tryptophan catabolism. Science translational medicine 5, 193ra91 (Jul 10, 2013).
24. Zhou et al. 2011 PHAST: A Fast Phage Search Tool. Nucl. Acids Res. 39 suppl. 2: W347-W352
25. U.S. Patent No. 9,415,079. Composition for inducing proliferation or accumulation of regulatory T cells.

### Examples

### Example 1: Assessing pathogenic and colitogenic potential of isolated Klebsiella pneumoniae 2H7 ("KP2") and Enterobacter aerogenes 11E12 strains ("KP3")

### Klebsiella pneumoniae 2H7 strain

The candidate pathogenic *Klebsiella pneumoniae* strain ID 2H7 ("KP2") was isolated from a human saliva sample. Human saliva samples were collected at the Hospital of University of the Ryukyus using a study protocol as described in Said et al. The source of KP2 strain was saliva sample from a patient with Crohn's Disease [IBD121, 52-year-old Japanese man, IOIBD score 1 (remission-phase), "CD patient #2, CD#2"]. Saliva samples from patients with CD [IBD029, 50-year-old Japanese man, IOIBD score 3 (active-phase), "CD patient #1, CD#1"; IBD121, 52-year-old Japanese man, IOIBD score 1 (remission-phase), "CD patient #2, CD#2"], UC (IBD096, 23-year-old Japanese woman, UC-DAI mild, "UC patient #1, UC#1"; IBD118, 65-year-old Japanese man, UC-DAI moderate, "UC patient #2, UC#2") and from healthy donors (H11, 22-year-old Japanese woman; H17, 23-year-old Japanese man) were selected as representatives of each group (healthy, CD, and UC) on the basis of the principal coordinate analysis of 16S rRNA sequencing data of saliva microbiota. Saliva samples were suspended in equal volume (w/v) of PBS containing 40% glycerol, snap-frozen in liquid nitrogen, and stored at -80°C until use. The frozen stocks were thawed and orally inoculated into GF mice (250 µL per head).

Saliva samples from the patients with Crohn's disease were first transplanted into C57BL/6 (B6) germ-free (GF) mice by gavage. The mice were purchased from Sankyo Laboratories Japan and maintained under GF conditions. Each group of mice was housed in separate gnotobiotic isolators for 3 weeks, at which time small intestinal and colonic lamina propria (LP) immune cells were examined.

To isolate LP lymphocytes, small and large intestines were collected and opened longitudinally, washed with phosphate buffered saline pH 7.4 (PBS) to remove all luminal contents, and shaken in Hanks' balanced salt solution (HBSS) containing 5 mM ethylenediaminetetraacetic acid (EDTA) for 20 min at 37°C. After epithelial cells and muscle layers were removed using forceps, the LP layers were cut into small pieces and incubated with RPMI1640 containing 4% fetal bovine serum, 0.5 mg/mL collagenase D, 0.5 mg/mL dispase, and 40 µg/mL DNase I for 1 h at 37°C in a shaking water bath. The digested tissues were washed with HBSS containing 5 mM EDTA, resuspended in 5 mL of 40% Percoll, and overlaid on 2.5 mL of 80% Percoll in a 15-mL Falcon tube. Percoll gradient separation was performed by centrifugation at 800 ×g for 20 min at 25°C. Lymphocytes were collected from the interface of the Percoll gradient and suspended in ice-cold PBS and stimulated with 50 ng/mL phorbol myristate acetate (PMA) and 750 ng/mL ionomycin in the presence of Golgistop at 37°C for 4 h. After dead cells were labeled using the LIVE/DEAD fixable stain kit, the cells were permeabilized and stained with anti-CD3, CD4, TCRβ, IL-17A, IPN-γ, T-Bet, and RORγt using the Foxp3 staining buffer set. All data were collected on a BD LSRFortessa or FACSAria III and analyzed with Flowjo software. CD4⁺ T cells were defined as a CD4⁺ TCRβ⁺ subset within the live lymphocyte gate.

In mice receiving a saliva sample from CD patient #1 (GF+CD#1 mice), there were no significant changes in the intestinal immune cell characteristics. In contrast, in the group that received a saliva sample from CD patient #2 (GF+CD#2 mice), there was a marked accumulation of interferon-γ (IPN-γ)+CD4+ T cells (TH1 cells) in the intestinal LP. (Figure 1A). The community composition of the saliva microbiota before administration into GF mice and consequent fecal microbiota of the colonized animals (ex-GF mice) were compared by 16S rRNA gene sequencing. To this end, the cecal contents from mice were suspended in 10 mL of Tris-EDTA buffer containing 10 mM Tris-HCl (pH 8.0) and 10 mM EDTA containing 100 µg/mL RNase A, and lysozyme was added to a final concentration of 15 mg/mL. The suspension was incubated for 1 h at 37°C with gentle mixing. Purified achromopeptidase was added to a final concentration of 2,000 unit/mL, and the sample was further incubated for 30 min at 37°C. Then, sodium dodecyl sulfate (final concentration, 1%) was added to the suspension and mixed well. Subsequently, proteinase K was added (final concentration, 1 mg/mL) to the suspension, and the mixture was incubated for 1 h at 55°C. High-molecular mass DNA was extracted by phenol:chloroform:isoamyl alcohol (25:24:1), precipitated by polyethylene glycol 8000 (PEG 8000), washed with 70% ethanol, and resuspended in TE. PCR was performed using Ex Taq and (1) the 454 primer A (5'-CCATCTCATCCCTGCGTGTCTCCGACTCAG (454 adaptor sequence) + barcode (10 bases) + AGRGTTTGATYMTGGCTCAG-3' (27Fmod)) and (2) the 454 primer B (5'-CCTATCCCCTGTGTGCCTTGGCAGTCTCAG (454 adaptor sequence) + TGCTGCCTCCCGTAGGAGT-3' (338R)) to the V1-V2 region of the 16S rRNA gene. Amplicons generated from each sample (-330 bp) were subsequently purified using AMPure XP. DNA was quantified using a Quant-iT Picogreen dsDNA assay kit and a TBS-380 Mini-Fluorometer. Then, the amplified DNA was used as template for 454 GS Junior pyrosequencing using GS Junior Titanium emPCR Kit-Lib-L, GS Junior Titanium Sequencing Kit, and GS Junior Titanium PicoTiterPlate Kit according to the manufacturer's instructions. Quality filter-passed reads were obtained by removing reads that did not have both primer sequences, had an average quality value of <25, and were possibly chimeric. Of the filter-passed reads, 3,000 reads were used after trimming off both primer sequences for each sample and subjected to OTU analysis with the cutoff similarity of 96% identity. Representative sequences from each OTU were blasted to databases from the RDP and reference genome database constructed from publicly available genome sequences in NCBI and Human Microbiome Project databases.

Although the saliva samples of both patients had similar microbial communities, the fecal microbiota compositions differed markedly between GF+CD#2 mice and GF+CD#1 mice. Most of the bacterial species observed in the fecal microbiota of ex-GF mice were found to be minor components of the salivary microbiota, suggesting that bacterial species that constitute a small fraction of the oral microbiota can colonize in the gut, and a further limited subset can induce the accumulation of intestinal TH1 cells.

To isolate TH1-cell-inducing bacteria in CD#2 sample, cecal content from GF+CD#2 mice was serially diluted with PBS and seeded onto nonselective agar plates (Schaedler, BHI, GAM, CM0151, BL, and TS). After culture under anaerobic conditions (80% N2, 10% H2, 10% CO₂) in an anaerobic chamber at 37°C for 2 or 4 days, individual colonies were picked, and DNA was extracted and subjected to sequencing of the 16S rRNA gene fragment. 224 colonies were picked for GF+CD#2 mice from the culture media with different colony appearances. Individual isolates in the culture collection were grouped into "strains" if they shared the 16S rRNA gene sequence with >96% identity. The resulting strain sequences were compared to those in the Ribosomal Database Project (RDP) database and to OTUs observed in cecal samples to determine closely related species or strains and corresponding OTUs.

Sequencing of the 16S rRNA gene revealed that these colonies comprised eight strains, including *Gemella, Bifidobacterium, Streptococcus, Escherichia, Fusobacterium, Veillonella, Anaerococcus,* and *Klebsiella.* The eight strains were cultured and introduced as a mixture (8-mix) into GF mice. To prepare the bacterial mixture, bacterial strains were individually grown in EG broth to confluence and mixed at equal volumes of medium. The mixture of isolates was orally administered to GF mice (approximately 1 × 10⁸ CFU of total bacteria in 150 µL medium per head). All mice receiving a given mixture of bacterial strains were maintained in a single gnotobiotic isolator. *K. pneumoniae* strains were grown at 37°C in Luria-Bertani (LB) broth or on LB agar plates.

In mice inoculated with 8-mix, efficient induction of TH1 cells was observed in the colonic LP, with a magnitude comparable to that observed in GF+CD#2 mice. Because *Fusobacterium* (Fu) and *Veillonella* (Ve) have been implicated in IBD pathogenesis (Gevers et al., 2014), mice were colonized with two strains (strain ID Fu-21f and Ve-2E1). Unexpectedly, however, colonization with Fu-21f and Ve-2E1 strains resulted in only marginal elevation of TH1-cell frequency. Whereas, surprisingly, oral administration with *Klebsiella pneumoniae* strain ID 2H7 ("KP2") alone induced TH1 cells, while a mixture of the remaining seven strains (7-mix) failed to do so, suggesting that the KP2 strain was the major contributor to the accumulation of TH1 cells observed in GF+CD#2 mice. (Figure 1B).

The colitogenic potential of KP2 was examined. KP2 did not induce any inflammatory changes in the intestine of wild-type (WT) hosts, either monocolonized in B6 GF mice or colonized in Amp-treated B6 SPF mice, despite inducing TH1 cells. Since the interplay between microbial and genetic factors contributes substantially to the pathogenesis of IBD, the influence of KP2 colonization in I110-/- B6 mice (Jackson Laboratories), which are genetically prone to colitis, was examined. GF I110-/- mice or Amp-treated SPF I110-/- mice were orally administered KP2 or Escherichia coli strain ID 2B1 (Ec-2B 1), which was also isolated from GF+CD#2 mice but had a weak ability to induce TH1 cells. Both *K*. *pneumoniae* and *E. coli* belong to Enterobacteriaceae, a family that has been implicated in IBD pathogenesis (Gevers et al., 2014). Histological analysis showed that KP2 induced more severe inflammation than Ec-2B 1 in the proximal colon of ex-GF or Amp-treated SPF I110-/- mice, suggesting that KP2 acts as a gut pathobiont by inducing the TH1 cell response. (Figure 1C). KP2 may also induce additional pro-inflammatory effects in a species-specific and host genotype-specific fashion.

### Enterobacter aerogenes 11E12 strain

The candidate pathogenic *Enterobacter aerogenes* strain ID 11E12 ("KP3") was isolated from human saliva samples. Human saliva samples were collected at the Hospital of University of the Ryukyus using a study protocol as described in Said et al. The source of KP3 strain was the saliva of a patient with Ulcerative Colitis [IBD118, 65-year-old Japanese man, UC-DAI moderate, "UC patient 2, UC#2"]. Tested saliva samples were suspended in equal volume (w/v) of PBS containing 40% glycerol, snap-frozen in liquid nitrogen, and stored at -80°C until use. The frozen stocks were thawed and orally inoculated into GF mice (250 µL per head) as described for KP2.

Saliva samples from the two patients with active ulcerative colitis (UC patient #1 and UC patient #2) were administered to GF WT B6 mice and analyzed as described for *Klebsiella pneumoniae* KP2 strain. Marked accumulation of TH1 cells was observed in the colonic LP of mice inoculated with a saliva sample from UC patient #2 (GF+UC#2 mice), and the level was comparable to that observed in GF+CD#2 mice. (Figure 2A).

To isolate strains responsible for the observed TH1 cell induction in the UC#2 sample, cecal content was collected from GF+UC#2 mice and cultured *in vitro* as described for culturing of CD#2 sample. 13 strains were isolated and roughly represented the microbiota configuration of GF+UC#2 mice. The oral administration of a mixture of 13 strains (13-mix) in GF mice replicated the phenotype observed in GF+UC#2 mice in terms of colonic TH1-cell induction. Among 13 strains, *Enterococcus faecium* strain ID 11A1 (Ef-11A1) and *Klebsiella aeromobilis* 11E12 ("KP3") had been reported to be important multidrug-resistant pathobionts and implicated in IBD pathogenesis (Taur et al., 2013; Davin-Regli et al., 2015; Mondot et al., 2011; Diene et al., 2013). GF mice were gavaged with either Ef-11A1, KP3, or a mixture of the 11 other strains (11-mix). KP3 induced a marked accumulation of TH1 cells in the colon at a level comparable to that observed in GF+13-mix and GF+UC#2 mice, whereas Ef-11A1 or 11-mix failed to do so. Therefore, KP3 was likely associated with the induction of TH1 cells, although KP3 was a minor member of the gut microbiota in GF+UC#2 mice. (Figure 2B).

Next, the colitogenic potential of the isolated KP3 strain was assessed in *Il10*^{*-*/*-*}mice as described for KP2. KP3 colonization resulted in inflammation in *Il10*^{*-*/*-*} mice, suggesting that the oral-derived *K. aeromobilis* strain, may also act as a pathobiont in the intestine. (Figure 2C).

### Example 2: Sample sourcing and processing for bacteriophage.

Sewage was obtained from the National Virology Center at Sheba Hospital. Dental sewage was collected from the dental clinic at Poria Hospital. Fecal samples were provided from healthy donors at Ichilov Hospital.

Batches of sewage comprised of 5-6 samples obtained from different places at different times were centrifuged and the supernatant was filtered (Merck Millipore glass fiber prefilters APFD, APFB followed by Express plus PES 47mm disks 0.45µm filters) using a vacuum filtration system. During this process 400 mL of 5-6 samples were mixed into one pool. The pooled sewage sample mix was concentrated by the Pellicon filter system (from 2L to 20mL). Concentrated sewage sample is filtered with 0.45µM filter, passed to barcoded tube and stored at 4°C. Thus, each sewage phage isolate in the bank is comprised of phage from 5-6 samples of different geographical origins.

Dental sewage samples of 4-5 different donors were processed in a similar way to regular sewage, except that the starting volume of batches was <400mL. Concentrated dental sewage sample is filtered with 0.45u filter, passed to barcoded tube and stored at 4°C.

Fecal samples were processed individually as follows: 5g of each fecal sample of an individual donor was weighed and inserted into a BagMixer plastic bag together with SM buffer (50mM Tris, 200mM NaCl, 10mM MgSO4, 0.01% Gelatin). The fecal matter was re-suspended by 2 cycles of 2 minutes in a BagMixer 400P stomacher. The resulting suspension was centrifuged and the remaining supernatant was filtered by 0.45 µm filter to remove the bacterial fraction and kept at 4°C.

### Example 3: Screening environmental and clinical samples for phage that recognize K. pneumoniae clinical strains.

KP2 and KP3 were isolated and sequenced as described herein to produce reference genomes. Subsequent alignments were performed with Illumina reads using bowtie2 to the reference genome. Overall alignments of >98% confirmed that subsequent strains were pure. An in-silico 16S PCR was run on the KP2 and KP3 genomes resulting in amplicons that both NCBI BLAST (Altschul et al. 1997) and the Ribosomal Database Project (Cole et al. 2014) matched to the 16S of *Klebsiella pneumoniae* and *Enterobacter aerogenes* respectively. The assignment of KP3 to *Enterobacter aerogenes* was further confirmed by colony mass spectrometry.

10 dental sewage samples, 41 sewage samples and 70 fecal samples were used to assemble mini-mixes or 3-sample mixes (total 39 mixes). Screening was performed by testing 10 µL of each mix on both KP2 and KP3 bacterial lawns, in 48 well plates by drop plaque assay (Bacteriophages methods and protocols MRJ Clokie AM Kropinsky). Plates were incubated for 2-3 hrs (37°C) in aerobic conditions, after which plaques became visible on the bacterial lawns. By this procedure plaques were detected in 1/39 wells on KP2, and plaques in 11/39 wells on KP3 as shown in Table 2. Plaques observed on KP3 were isolated as follows: from 5/11 wells one plaque each was picked, from 4/11 wells two different plaques were picked by morphology, from 2/11 wells three different plaques were picked by morphology. A single plaque was isolated for KP2. Plaques were picked up (using a sterile 20 µL tip) into phage buffer (Tris-HCL pH 7.5 50mM, NaCl 100mM, MgCl₂^{∗}6H₂O 5mM, MnCl₂^{∗}4H₂O 0.1mM in DDW ) and re-isolated for a total of 3 rounds (See table 2).

**Table 2. KP2 and KP3 phage obtained from environmental samples.**

| **Phage name** | **Host** | **Mix no.** | **Sample no. (each sample is a mixture of 5)** |
|---|---|---|---|
| KP2W-P0106 | KP2 | 5 | 1-3 |
| KP3W-P0109 | KP3 | 5 | 1-3 |
| KP3W-P0201 | KP3 | 6 | 4- 6 |
| KP3W-S0101 | | | |
| KP3W-P0202 | KP3 | 7 | 7- 9 |
| KP3W-P0103 | | | |
| KP3W-P0112 | KP3 | 8 | 10- 12 |
| KP3W-P0105 | KP3 | 11 | 19- 21 |
| KP3W-P0102 | | | |
| KP3W-P0101 | KP3 | 12 | 22- 24 |
| KP3W-P0108 | KP3 | 13 | 25- 27 |
| KP3W-P0110 | KP3 | 15 | 31- 34 |
| KP3W-P0113 | | | |
| KP3W-P0114 | | | |
| KP3W-P0106 | KP3 | 16 | 35- 37 |
| KP3W-P0203 | | | |
| KP3W-S0102 | | | |
| KP3W-P0104 | KP3 | 17 | 38-40 |
| KP3W-P0107 | | | |
| KP3W-P0111 | KP3 | 18 | 41 |

To enlarge the pool of phage that recognize KP2, the environmental and clinical samples were enriched for the desired phage as follows: 10 dental sewage samples, 41 sewage samples and 70 fecal samples were used to assemble 6-sample mixes (total 21 mixes). Enrichments were performed by culturing KP2 in 21×4 mL culture tubes plus 100 µL of each mix, and incubating for 3 hrs at 37°C. During this period any phage that infects the KP2 would be amplified to enable easier detection by plaque assay. The KP2 phage found via enrichments may be amplified wild-type phage that were present in the original samples or phage that have acquired specific mutations allowing more efficient infections during the directed enrichment process.

Following the incubation, the tubes were centrifuged, supernatant was filtered by 0.45 µm syringe filter and tubes were kept at 4°C. Screening of the enriched environmental samples was performed by testing 10 µL of each on KP2 bacterial lawns, in 48 well plates by drop plaque assay. Plate was incubated for 2-3 hrs at 37°C, at aerobic conditions, after which plaques became visible on the bacterial lawn. By this procedure plaques were detected in 4/21 enrichments as shown in Table 3. From 3/21 wells one plaque each was picked, and from 1/21 wells two different plaques were picked by morphology. Plaques were picked up (using a sterile 20 µL tip) into phage buffer and re-isolated for a total of 3 rounds.

**Table 3. Additional KP2 phage obtained by enrichments of environmental and clinical samples. SWG - sewage, fecal - fecal clinical samples obtained from healthy of IBD donors.**

| **Name** | **Host** | **Enrichment source** |
|---|---|---|
| KP2W-M0104 | KP2 | SWG(4-6) +fecal(13-15) |
| KP2W-P0101 | KP2 | SWG(31-34) +fecal (41-44) |
| KP2W-P0102 | KP2 | SWG(35-37) +fecal (45-47) |
| KP2W-P0103 | KP2 | SWG(35-37) +fecal (45-47) |
| KP2W-P0104 | KP2 | SWG(38-40) +fecal (48-50) |

### Example 4: Phage amplification and determination of phage titers.

Out of the 25 phages (6 phage isolated on KP2 plus 19 phage isolated on KP3), part were amplified from liquid broth whereas the rest were amplified from soft agar by double agar overlay plaque assay. Amplification from liquid broth was performed by diluting 50 µL of isolated phage sample into 4 mL log phase culture at OD=1.7, or into 4 mL log phase culture at OD=0.5, and incubating at 37°C, overnight. Tubes were centrifuged, the supernatant was filtered by 0.45 µm syringe filter, and 1 mM of the divalent ions Ca²⁺ and Mg²⁺ were added.

Phage titers were determined by drop plaque assay as follows: host culture was prepared by inoculating 4 mL liquid BHIS (BACTO^{™} Brain Heart Infusion BHI, Yeast extract 0.5% and Resazurin) with 5-10 colonies of the host and incubating at 37°C, until OD was 1.7 (1.5-2 hrs). 100 µL of host culture were added to 4 mL of molten top agar (BHIS top agar: BHIS media, 0.4% Agarose) with divalent ions Ca²⁺ Mg²⁺ and dispensed (100 µL/well) to 48 well plate with underlay of bottom agar (BHIS bottom agar: BHIS media, 1.6% Agarose). Plate was incubated for 30 minutes (37°C), and then serial dilutions of phage sample were dropped (10 µL). Plate was incubated for 2-3 hrs before counting plaques (10-50 plaques) and determination of phage titer (number of plaques × 10 × reciprocal of counted dilution = PFU/mL).

Amplification of phage from soft agar was performed in cases where titers of phage that were produced from liquid BHIS were lower than 1×10⁹ PFU/mL. Host culture at OD=1.7, was diluted to OD=1, and aliquots of 100 µL diluted host culture were added to 100 µL of phage at a titer that is sufficient to result in production of confluent plaques, and incubated at room temperature for 15 minutes. Then each host-phage mixture was diluted with 3 mL soft molten agar, and poured on top of BHIS bottom agar. Plates were let to harden, and then incubated at 37°C for 2-3 hrs or until complete lysis. Plates were scraped from the top agar with a spreader and agar was transferred to sterile tubes with BHIS and 10 µL of chloroform. Tubes were allowed to stand for 30 minutes, to allow phages to elute from the soft agar. Tubes were centrifuged, and supernatant was filtered with 0.45 µM syringe filter to eliminate residual agar. Divalent ions Ca²⁺ Mg²⁺ at concentration of 1mM were added to tubes which were stored at 4°C.

### Example 5: Host range analysis of isolated phage.

Host range analysis for phage isolated on KP2 and KP3 strains, as well as for the commercial phage (ATCC 23356-B 1) was performed on six K. pneumoniae strains from ATCC: BAA-2552 (KP1), 23356 (KP4), 13882 (KP5), BAA-1705 (KP6), 700603 (KP7), 700721(KP8). Each phage was added (10 µL) to bacterial lawns of K. pneumoniae strains, in 48 well plates by drop assay. Plates were incubated for 2-3 hrs (37°C) in aerobic conditions, after which plaques become visible on the bacterial lawns. Plates with bacterial lawns of KP2 and KP3 and their respective phage served as positive control. Host range was done for each of the phage with two phage titers; 1×10⁶ PFU/mL and 1×10⁹ PFU/mL. As can be seen in Figure 3, where dark gray indicates susceptibility and gray indicates resistance, all phage exhibited high specificity for their hosts. This was the case for both titers tested. The exception to this observation was for phage KP2W-M0104 which infected 23356 strain at titer of 1×10⁹ PFU/mL, however no plaques were observed for KP2W-M0104 on 23356 at a lower titer of 1×10⁶ PFU/mL. Thus, phage isolated on KP2 and KP3 are each specific for their hosts, meaning that they recognize specific receptors/attachment sites expressed exclusively by these bacterial strains (KP2, KP3). Moreover, the commercial phage ATCC 23356-B 1 did not recognize neither KP2 nor KP3, but only the strain KP4 on which it was isolated, suggesting that the newly isolated phage have a novel function.

### Example 6: Growth curves of KP2, KP3, and mutant.

The growth curves of KP2 and KP3 strains were generated by monitoring OD at 600nm using a plate reader for strains cultured alone, in the presence of individual phage, or in the presence of a cocktail of all the phages available against each bacterial strain. Briefly, KP2 and KP3 strains were cultured in 4mL liquid BHIS to OD600=1.7. Then, cultures were diluted to OD600=0.2 and dispensed (0.2mL/well) into a 96 well plate. Each bacterial strain was tested in duplicate with respective individual phages (KP2 phages (n=6) and KP3 phages (n=19), or in the presence of a phage cocktail containing all respective phages in equal ratios and in a total number equivalent to that of individual phage alongside the phage-free controls. Phage titers were 1×10⁶ PFU/mL at a volume of 10µL/well of phage or phage cocktail. Wells were filled with Mineral oil (50µL/well) and the plate was sealed with an optical seal to avoid evaporation of sample during incubation. Plate was inserted to the robot plate reader (Freedom EVO 75, Tecan), plate was incubated (37°C) and read every 15 minutes for 22 hrs at OD=600 nm. Analysis of the results for KP2 strain are presented in Figure 4. KP2 growth curve without phage is illustrated by the black line, while the remaining lines present the growth curves of KP2 with a single phage as indicated. The dashed line represents KP2 growth curve in the presence of the cocktail of six phages. The KP2 growth curves generated in the presence of individual phages demonstrate the regrowth of resistant or appearance of mutant strains after 1-2 hrs. In contrast, the growth of KP2 in the presence of the cocktail displays appearance of resistant mutants after 9 hrs enabling significant improvement in a "time to mutant" parameter. Similar analysis performed for KP3 strain resulted in tripled "time to mutant" or even no appearance of mutants during the course of the study.

### Example 7: Creating KP2 mutants for the screening of additional phage

During the infection of the bacteria by a phage, mutant bacterial strains arise that are resistant to the phage. To target the resistant mutant strains the following strategy was employed: first, the mutant KP2 strains arising as a result of the incubation with the cocktail of the following phage KP2W-P0101, KP2W-P0102, KP2W-P0103, KP2W-P0104 and KP2W-M0104 (phage titers of 1×10⁶ PFU/mL) as well as the mutants arising against KP2W-P0104 phage were collected and characterized. To this end, KP2 strain was cultured in 4 mL BHIS, at 37°C with shaking to OD₆₀₀ₙₘ=1.5. Then, culture was diluted with liquid BHIS to OD=0.2 and dispensed (200 µL/well) into 96 wells plate. A volume of 10 µL of KP2W-P0104 phage was added to create MKP2_2161_1 mutant and 10 µL of a cocktail of the phage described above (at equal amounts) were added to create MKP2_coc_1 mutant. Each well was covered with mineral oil (50 µL/well) and sealed with a tape to avoid evaporation of sample. Absorbance (600nm) was monitored in plate reader every 15 min for 24 hrs. Analysis of the growth curves of KP2 alone, or KP2 infected with KP2W-P0104, or with all six phage, revealed mutant candidates. The contents of the wells containing mutant candidate were plated on BHIS agar plates and incubated overnight at 37°C. Next, single colonies were isolated and propagated in liquid BHIS. The candidates were verified as *Klebsiella pneumoniae* by 16S pyrosequencing followed by confirmation of resistance to the respective phages by drop assay and whole genome sequencing. MKP2_2161_1 was found to be resistant to all phage isolates but KP2W-M0104, and MKP2_coc_1 was found to be resistant to all phages tested. The two mutants MKP2_2161_1 and MKP2_Coc_1 were used in phage selections to further expand our phage pool.

### Example 8: Enrichments with MKP2_2161_1 and MKP2_coc_1

To isolate phages capable of infecting the resistant KP2 mutants, MKP2_2161_1 and MKP2_coc_1, the following environmental and clinical samples were enriched on MKP2_2161_1 and MKP2_coc_1: 19 dental sewage, 58 sewage and 100 fecal samples were used to assemble 7-9 sample mixes (total 19 mixes). Enrichments were performed by culturing each of the mutants (MKP2_2161_1 and MKP2_Coc_1) with each of the 19 mixes in 19x4 mL culture tubes plus 150 µL of each mix, for 3 hrs at 37°C. Following the incubation, the tubes were centrifuged, supernatants were filtered by 0.45µm syringe filter and kept at 4°C. Screening of the enriched samples was performed by testing 10 µL of each enriched sample on KP2, MKP2_2161_1 and MKP2_Coc_1 bacterial lawns, in 48 well plates by drop plaque assay. Plates were incubated for 2-3 hrs at 37°C, at aerobic conditions, after which plaques became visible on bacterial lawn. This procedure yielded plaques in 8/19 enrichments as shown in the Table 4. Enrichments on MKP2_2161_1 tested on MKP2_2161_1 lawn resulted in 9 phage and enrichments on tested on MKP2_Coc_1 resulted in 17 phage from 8 different sources. Enrichments on MKP2_Coc_1 were tested for infectivity on MKP2_2161_1 and MKP2_Coc_1 lawns and yielded 10 plaques from 8 different sources. Enrichments on MKP2_2161_1 tested on KP2 lawn and enrichments on MKP2_Coc_1 tested on KP2 lawn, resulted in 10 plaques from 6 different sources (Table 4). Plaques were picked up using a sterile 20 µL tip into phage buffer and re-isolated for a total of 3 rounds.

**Table 4: Phage against KP mutants obtained from enrichments of phage isolates.**

| Enrichments/re-isolation (either one or two rounds, or no (0)) on MKP2_2161_1 resulted in 9 phage and enrichments/re-isolation (ranging from one to three rounds, or no (0)) on MKP2_Coc_1 resulted in 17 phage from 8 different sources. Enrichment were tested on MKP2_2161_1, MKP2_Coc_1 and on KP2 lawns. SWG - sewage, DSWG - dental sewage, fecal - fecal clinical samples obtained from either healthy or IBD donors. | | | | |
|---|---|---|---|---|
| Phage name | Mini mix no | Source and sample no. (each sample is a mixture of 5) | MKP2_2161_1 enrichment rounds | MKP2_coc_1 enrichment rounds |
| KP2W-M0102 | 3 | 19 (DSWG) | 1 | 2 |
| KP2M-M0109 | | 1-6 (SWG) | | |
| KP2M-P0204 | | | | |
| KP2W-M0103 | 4 | 7-15 (SWG) | 2 | 2 |
| KP2M-M0105 | | | | |
| KP2W-P0202 | | | | |
| KP2M-P0207 | | | | |
| KP2M-M0110 | 5 | 16-24 (SWG) | 1 | 2 |
| KP2M-P0203 | | | | |
| KP2W-M0106 | | | | |
| KP2M-M0111 | 6 | 25-34 (SWG) | 0 | 2 |
| KP2M-P0206 | | | | |
| KP2W-P0109 | 7 | 35-42 (SWG) | 1 | 3 |
| KP2W-P0107 | | | | |
| KP2M-M0112 | | | | |
| KP2W-P0105 | 8 | 44-52 (SWG) | 2 | 2 |
| KP2W-P0108 | | | | |
| KP2M-P0201 | | | | |
| KP2M-P0212 | | | | |
| KP2M-P0208 | 9 | 53-58 (SWG) | 2 | 3 |
| KP2M-P0209 | | | | |
| KP2M-P0210 | | | | |
| KP2W-M0107 | | 1-3 (Fecal) | | |
| KP2M-M0108 | | | | |
| KP2M-P0211 | 15 | 51-56 (Fecal) | 0 | 1 |

### Example 9: Host range analysis of newly isolated phages

Host range analysis for phage isolated on MKP2_2161_1, MKP2_Coc_1 and on KP2 strains, was performed on four additional KP mutants; MKP2_2161_C (produced against KP2W-P0104), MKP2_251_B, MKP2_251_C and MKP2_251_D (produced against KP2W-M0104) and on KP4 strain. Each of the phage were added (10 µL) to bacterial lawns of the different K. pneumoniae strains, in 48 well plates by drop assay. Plates were incubated for 2-3 hrs (37°C) in aerobic conditions, after which plaques become visible on the bacterial lawns. Plates with bacterial lawns of MKP2_2161_1, MKP2_Coc_1 and KP2 and their respective phage served as positive control. Host range was done for each of the phage with two phage titers; 1×10⁶ PFU/mL and 1×10⁹ PFU/mL. Table 5, Table 6, and Table 7 show phage that were isolated on MKP2_2161_1 and MKP2_Coc_1 did not infect KP2. However, phage that were isolated on KP2 infected part or all KP2 mutant strains as well as the commercial strain KP4.

**Table 5. Characterization of isolated phage.**

| Phage | Isolated on | Size (Kb) | GC % | # genes | VIRFAM analysis | Most similar to |
|---|---|---|---|---|---|---|
| KP3W-P0101 | KP3 | 40.2 | 52.74% | 50 | Podoviridae of Type3 | phiYeO3-12 / T3 / T7 |
| KP3W-P0102 | KP3 | 40.6 | 52.93% | 48 | | |
| KP3W-P0103 | KP3 | 40.7 | 52.93% | 48 | | |
| KP3W-P0104 | KP3 | 41.4 | 52.83% | 49 | | |
| KP3W-P0105 | KP3 | 40.3 | 52.98% | 47 | | |
| KP3W-P0106 | KP3 | 40.7 | 52.45% | 49 | | |
| KP3W-P0107 | KP3 | 41.3 | 52.32% | 52 | | |
| KP3W-P0108 | KP3 | 41 | 52.41% | 53 | | |
| KP3W-P0109 | KP3 | 41.1 | 52.39% | 53 | | |
| KP3W-P0110 | KP3 | 41.2 | 52.59% | 52 | | |
| KP3W-P0111 | KP3 | 40.8 | 52.44% | 51 | | |
| KP3W-P0112 | KP3 | 40.7 | 52.42% | 50 | | |
| KP3W-P0113 | KP3 | 40.7 | 52.46% | 50 | | |
| KP3W-P0114 | KP3 | 40.4 | 52.34% | 47 | | |
| KP3W-P0201 | KP3 | 40.6 | 51.64% | 49 | | |
| KP3W-P0202 | KP3 | 40.4 | 51.69% | 50 | | |
| KP3W-P0203 | KP3 | 40.6 | 51.58% | 48 | | |
| KP3W-S0101 | KP3 | 51.7 | 51.71% | 81 | Siphoviridae of Type1 | T1 / TLS / RTP |
| KP3W-S0102 | KP3 | 50.4 | 51.31% | 73 | | |
| KP2W-P0101 | KP2 | 40.3 | 50.16% | 51 | Podoviridae of Type3 | phiYeO3-12 / T3 / T7 |
| KP2W-P0102 | KP2 | 40.4 | 50.16% | 51 | | |
| KP2W-P0103 | KP2 | 40.4 | 50.16% | 51 | | |
| KP2W-P0104 | KP2 | 38.2 | 50.92% | 45 | | |
| KP2W-P0105 | KP2 | 39.1 | 50.79% | 46 | | |
| KP2W-P0106 | KP2 | 39.4 | 50.73% | 48 | | |
| KP2W-P0107 | KP2 | 39 | 50.67% | 47 | | |
| KP2W-P0108 | KP2 | 39 | 50.66% | 47 | | |
| KP2W-P0109 | KP2 | 39 | 50.69% | 47 | | |
| KP2M-P0201 | KP2-Coc-M | 44.2 | 54.28% | 53 | | |
| KP2W-P0202 | KP2 | 43.8 | 54.13% | 56 | | |
| KP2M-P0203 | KP2-Coc-M | 43.8 | 54.15% | 54 | | |
| KP2M-P0204 | KP2-Coc-M | 43.5 | 54.19% | 53 | | |
| KP2M-P0205 | KP2-Coc-M | 43.9 | 54.10% | 54 | | |
| KP2M-P0206 | KP2-Coc-M | 44.8 | 53.96% | 56 | | |
| KP2M-P0207 | KP2-Coc-M | 43.2 | 54.21% | 53 | | |
| KP2M-P0208 | MKP2_2161_1 | 44.8 | 54.14% | 55 | | |
| KP2M-P0209 | KP2-Coc-M | 44.8 | 54.12% | 56 | | |
| KP2M-P0210 | KP2-Coc-M | 44.8 | 54.14% | 57 | | |
| KP2M-P0211 | KP2-Coc-M | 43.6 | 54.16% | 54 | | |
| KP2M-P0212 | KP2-Coc-M | 43.6 | 54.13% | 55 | | |
| KP2W-M0102 | KP2 | 178.1 | 44.89% | 274 | Myoviridae of Type2 | RB43 / T4 |
| KP2W-M0103 | KP2 | 179.7 | 44.90% | 280 | | |
| KP2W-M0104 | KP2 | 178.4 | 44.92% | 273 | | |
| KP2M-M0105 | MKP2_2161_1 | 178.9 | 44.90% | 276 | | |
| KP2W-M0106 | KP2 | 179 | 44.67% | 273 | | |
| KP2W-M0107 | KP2 | 178.1 | 44.84% | 270 | | |
| KP2M-M0108 | MKP2_2161_1 | 177.8 | 44.85% | 267 | | |
| KP2M-M0109 | MKP2_2161_1 | 178.6 | 44.76% | 270 | | |
| KP2M-M0110 | MKP2_2161_1 | 177.5 | 44.78% | 267 | | |
| KP2M-M0111 | MKP2_2161_1 | 178.8 | 44.72% | 275 | | |
| KP2M-M0112 | MKP2_2161_1 | 179.5 | 44.54% | 267 | | |

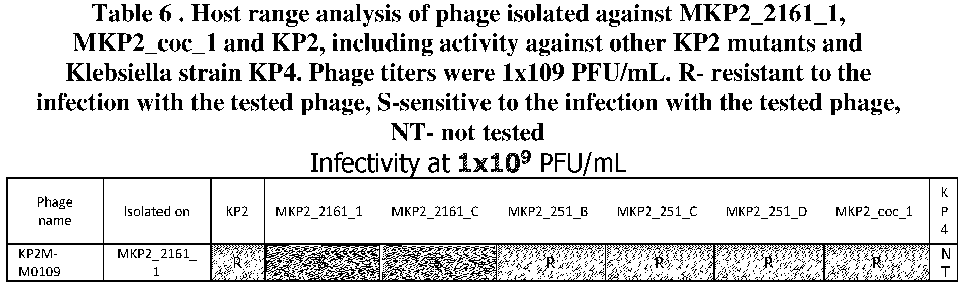

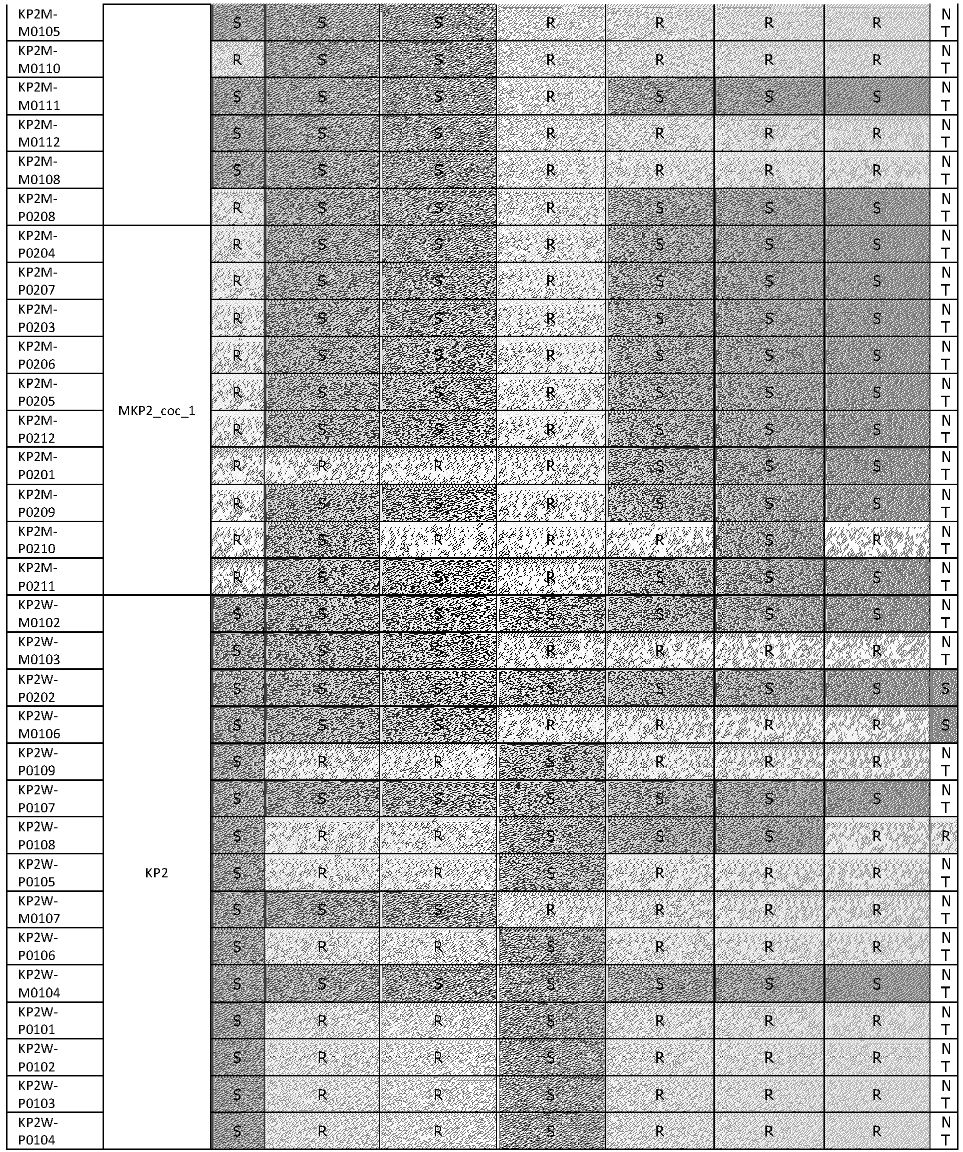

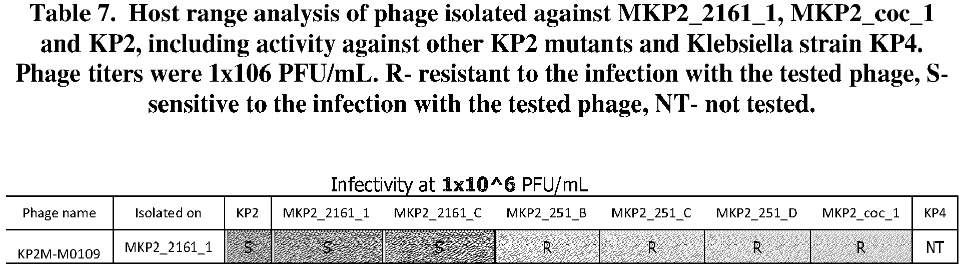

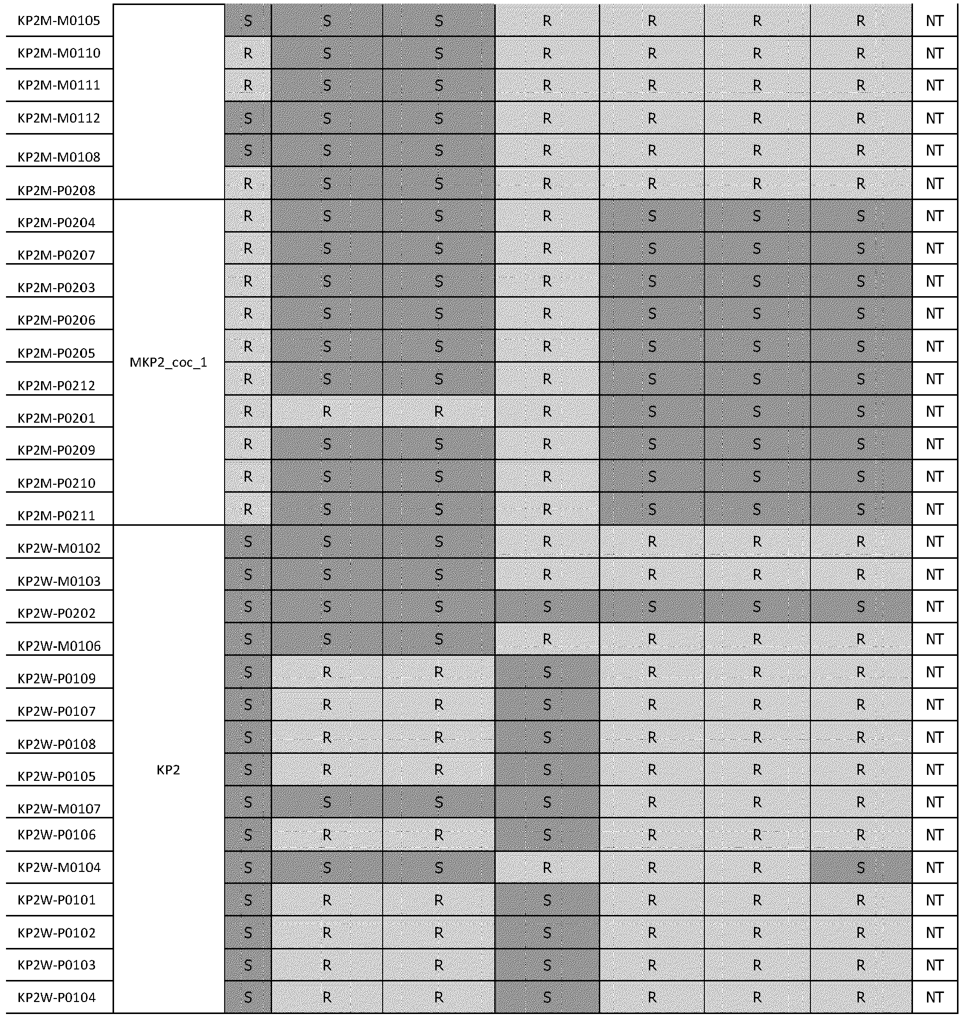

### Example 10: Phage DNA extraction and sequencing.

Phage DNA was extracted from 0.2 mL of phage at least 1×10⁹ PFU/mL using the QIAGEN QIAamp DNA Mini Kit and the following protocol. After extraction, Illumina libraries were created following the protocol of Baym et al. (2015) and sequenced using paired end 40bp reads with an average coverage of -250 reads per position.

### Example 11: Phage DNA assembly and analysis.

Reads were assembled using SPAdes 3.9.0 and annotated using PHAST (You Zhou et al. 2011). Phage structural genes were analyzed using VIRFAM (Lopes A, et al. Automated detection and classification of head-to-tail connection proteins in bacteriophages, BMC Genomics. 2014 Nov 27;15:1027) which clustered them into 3 different families: Myoviridae of Type 2 (such as known phages T4 or RB43), Podoviridae of Type 3 (such as known phages phiYeO3-12, T3 or T7) and Siphoviridae of Type 1 (such as known phages T1, TLS or RTP).

As can be seen in Table 5, the majority of phages isolated against KP3 strain belong to the Podoviridae family type 3 with genomes ~40kb, and only two phages belong to Siphoviridae family with genome size ~50kb. All phages but five isolated against KP2 strain belong to Podoviridae family type 3 as well as all phages isolated against KP2 mutant KP2-Coc-M (also referred to as MKP2-Coc_1) and one phage isolated against mutant KP2 MKP2-2161_1. Five phages isolated against KP2 and all phages but one isolated against mutant KP2 MKP2-2161_1 belong to Myoviridae family with a genome ~180kb genome, four times larger than the genomes of the Podoviridae.

As can be seen in Fig. 5, the Podoviruses that recognize KP2 cluster into two clusters with over 83% and 85% percent identity among them. Myoviruses that recognize KP2 cluster in a separate cluster with >86% homology.

The Podoviruses against KP3 cluster into two separate blocks (Figure 6) with >78% and >96% identity respectively and the percent of homology between the members from the two different clusters ranges from 38% to slightly over 50%. The Siphoviruses form a distinct cluster with >88% identity between the two.

Even though better-known phage (e.g., T4, T7 and T1) belonging to the same classification groups as our phage are usually known to be virulent (lytic-only) phage, the phage sequences were annotated using PHAST (Zhou et al. 2011.) to detect specific genes relating to lysogeny. The screening revealed no integrases, recombinases or other genes known to take part in the lysogenic cycle, suggesting that the phage were lytic-only as well, as required of phage for therapeutic purposes. PCR analysis of the two bacterial strains KP2 and KP3 with phage primers did not yield any PCR products.

### Example 12. In vivo testing.

The efficacy of the phage treatment is assessed. An *in vivo* platform is used to test 1) the colonization of virulent strains of *Klebsiella pneumoniae* in the gastrointestinal tract and 2) their eradication by the phage therapy. In some embodiments, a phage cocktail comprising several phages against a bacterial strain is tested. In some embodiments, the one or more of the phages in the phage cocktail delay the appearance of bacterial mutants and/or target bacterial mutants. In some embodiments, the *in vivo* platform may be used to test for colonization of gastrointestinal tract, e.g., with pathogenic strains of KP2. In some embodiments, the *in vivo* platform may be used to compare the inoculation efficiency following single and double administration of the KP2 strain pre-treated with two different antibiotic cocktails to induce colonization. Animals are inoculated with a bacterial culture and treated with a phage or a phage cocktail as described in Table 8. Bacterial burden and inflammation are assessed as described in Table 9.

**Table 8. Experimental procedure.**

| | |
|---|---|
| Mouse strain: | C57BL/6, 8 weeks, male |
| Conditions: | SPF for KP2 |
| Study group: | n=8 (housed together after SPF verification), n=8 (housed in individual SPF cages) |
| Antibiotic treatment: | Treatment 1 (group 1-3): continuous administration of the cocktail of ampicillin 200 mg/L, tylosin at 500 mg/L and kanamycin 500mg/L in drinking water starting 4 days prior to colonization |
| | |
| | Treatment 2 (group 4-6): continuous administration of the cocktail of ampicillin, vancomycin, metronidazole, kanamycin at 1 g/L on days -16 - -3, continuous administration of kanamycin at 500mg/L in drinking water starting 2 days prior to colonization |
| Pathogen: | Klebsiella pneumoniae KP2 strain cultured to logarithmic phase |
| Colonization protocol: | 10⁸ CFU/animal by gavage in 200 µL PBS, single administration on day 0 (day 5 of the antibiotic treatment), or multiple dosing on day 0 and 1 (day 5 and day 6 of the antibiotic treatment) |
| In-life phase duration: | 15 - 27 days |
| Sacrifice schedule: | Day 10 |
| Readouts: | To assess the degree of Klebsiella colonization throughout the development, the following readouts are used: |
| | 1. Whole-body and (intestine) imaging using the IVIS system on days -1, 1, 2, 3, 4, 7, 8, 9, 10 (10 intestine) |
| | 2. Assessment of Klebsiella count in daily stool samples by plating on growth media and quantitative PCR using strain-specific primers |
| | 3. Assessment of Klebsiella count in intestinal contents and mucosa by plating on selective media and quantitative PCR using strain-specific primers on day 10 |
| | 4. Sacrifice: intestine histology, intestine cytokine mRNA, liver and spleen bacterial count |

**Table 9. Study design.**

| Group # | Group ID | Pre-treatment | Challenge | Endpoints | | |
|---|---|---|---|---|---|---|
| 1 (4) | Control, no pathogen | Antibiotic treatment 1 or 2 (see above) | Vehicle | 1. Whole body imaging on day 0 (3 hours after the colonization), 1, 2, 3, 4, 7, 8, 9, 10. | | |
| 2 (5) | Single administration | | 10⁸ day 0 | | | |
| | | | | 2. Overnight stool collection on day -4, | | |
| 3 (6) | Double administration | | 10⁸ day 0 | -2, 0, 1, 3, 5, 7, 9, 10. | | |
| | | | 10⁸ day 1 | | a. Stool plating on LB + KAN agar plates | |
| | | | | | b. qPCR on stool samples using KP2-specific primers | |
| | | | | | c. luminescence assessment | |
| | | | | 3. Sacrifice on day 10. | | |
| | | | | | a. Intestine harvesting | |
| | | | | | b. IVIS on the intestine | |
| | | | | | c. Segmentation of the intestine into large intestine, small intestine and cecum | |
| | | | | | d. Estimation of the KP2 burden in the contents of the intestinal segments by | |
| | | | | | | i. Stool plating on Klebsiella agar plates |
| | | | | | | ii. qPCR on stool samples using KP2-specific primers |
| | | | | | | iii. luminescence assessment |
| | | | | | e. Estimation of the KP2 burden in the mucosa of the intestinal segments using the BagMixer extraction protocol | |
| | | | | | | i. plating on Klebsiella agar plates |
| | | | | | | ii. qPCR on stool samples using KP2-specific primers |
| | | | | | | iii. luminescence assessment |
| | | | | f. extraction of mRNA using RNAlater kit to potentially assess cytokine mRNA count | | |
| | | | | g. embedding intestine sections in paraffin for potential histology | | |
| | | | | h. assessing bacterial burden in liver and spleen | | |

### Example 13. Isolation of KP2-like clinical strains.

A volume of 10 µL of stool suspended in buffer was cultured in 4 mL Peptone water (M028, Hylabs) overnight at 37°C, after which 10 µl of the culture was streaked onto MacConkey and Chromagar Orientation plates (Hylabs). Colonies with the ability to grow on the MacConkey plate and presenting a pink, mucoid colony morphology or colonies that appeared blue on the Chromagar plate were tested for motility and the ability to form indole gas using UMI agar tubes (TT-147, Hylabs). A PCR of the 16S rRNA region was performed for non-motile and non-indole producing colonies. The PCR products were sequenced in order to determine identity as *Klebsiella pneumoniae.* Positive isolations were identified as KP2-like using PCR for a 211bp unique region (fwd primer: 5'AGCACTAGCGGCTGTGGTAT3' and rev primer: 5'ACTTACTCGGGCCCTTGATT3'). *See, e.g.,* Atarashi et al., 2017.

Host range analysis for the KP2 phages was performed on 18 KP2-like clinical strains. Each of the phage were added to bacterial lawns of the different *K. pneumoniae* strains, in BHIS agar plates by drop assay as following: a starter culture of 4 mL BHIS was inoculated with 5-10 colonies from a plate. This culture was incubated at 37°C for 1.5-2 hours. A volume of 150 µL of this culture was mixed with 4 mL of BHIS top agar (pre-molten 0.4% agar BHIS supplemented with 1 mM Ca²⁺ and Mg²⁺ ions), and poured on a petri dish containing BHIS bottom agar (BHIS with 1.5% agarose). After solidifying at room temperature, the plates were incubated for 30 minutes at 37 degrees. At this stage, 5 µL from samples containing 10⁶ pfu/ml phage was dropped and left to absorb. Plates were incubated overnight at 37°C in aerobic conditions, after which plaques became visible on the bacterial lawns. Host range was performed for each of the phages at a titer of 1×10⁶ PFU/mL (Table X). For a phage that is able to form at least 10 plaques on a specific clinical line lawn, the bacterial host was determined as sensitive to that phage.

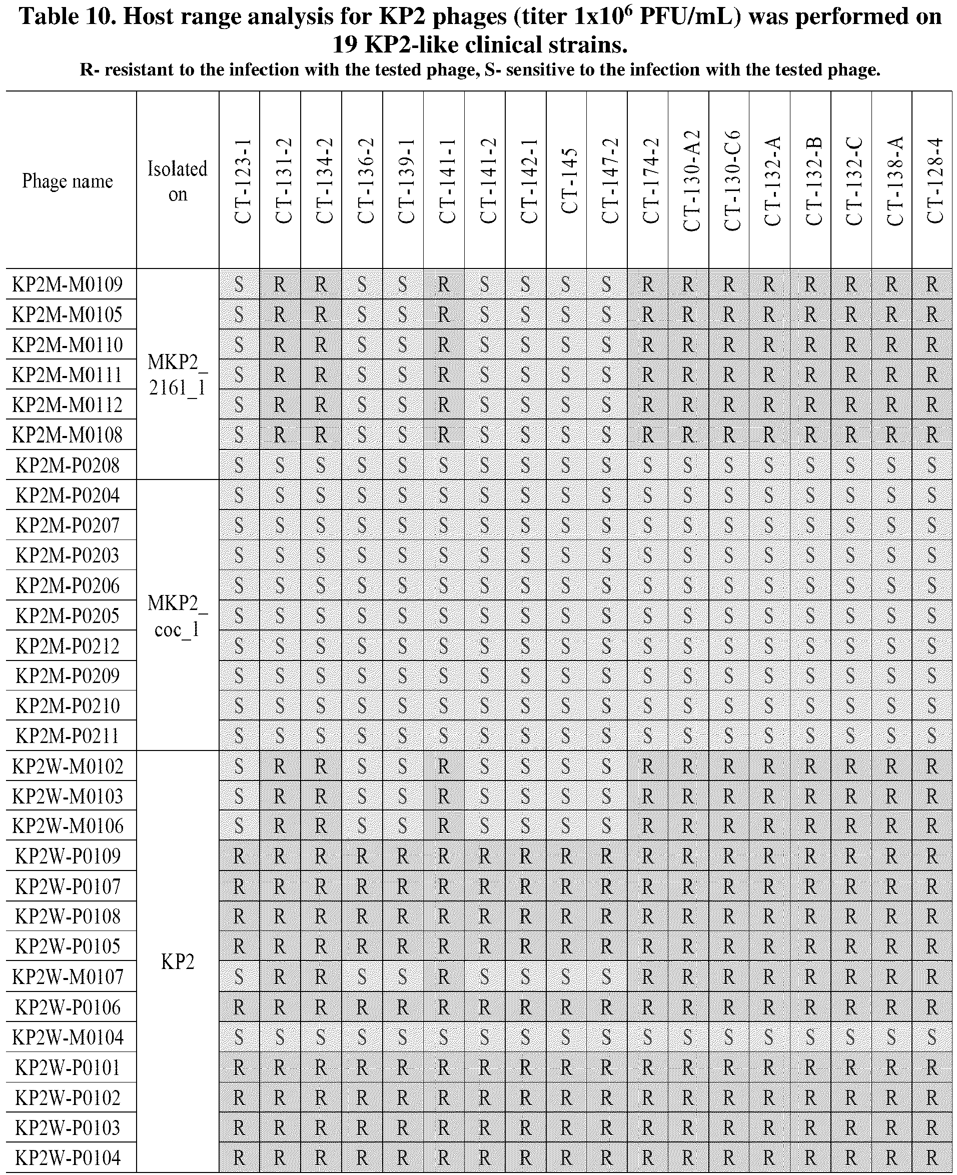

## Claims

1. A pharmaceutical composition comprising an isolated lytic bacteriophage having a genomic sequence at least 90 % homologous to one of the sequences as set forth in SEQ ID NO: 21-52, wherein said bacteriophage is capable of lytic infection of a *Klebsiella pneumoniae* 2H7 bacterial strain or a bacterial strain having greater than 85 % homology to said *Klebsiella pneumoniae* 2H7 bacterial strain, wherein the pharmaceutical composition is formulated for delivery to a mammalian intestine or formulated for delivery to a mammalian mouth.

2. The pharmaceutical composition of claim 1, wherein said lytic bacteriophage has a genomic sequence at least 95 % homologous to one of the sequences as set forth in SEQ ID NO: 21-52.

3. A pharmaceutical composition comprising an isolated lytic bacteriophage having a genomic sequence at least 98 % homologous to one of the sequences as set forth in SEQ ID NO: 1-19, wherein said bacteriophage is capable of lytic infection of a *Enterobacter aerogenes* 11E12 or a bacterial strain having greater than 85 % homology to said *Enterobacter aerogenes* 11E12 bacterial strain, wherein the pharmaceutical composition is formulated for delivery to a mammalian intestine or formulated for delivery to a mammalian mouth.

4. The pharmaceutical composition of claim 1 or 2, comprising at least two different lytic bacteriophages capable of lytic infection of said *Klebsiella pneumoniae* 2H7 bacterial strain or said bacterial strain having greater than 85 % homology to said *Klebsiella pneumoniae* 2H7 bacterial strain.

5. The pharmaceutical composition of claim 3, comprising at least two different lytic bacteriophages capable of lytic infection of said *Enterobacter aerogenes* 11E12 or a bacterial strain having greater than 85 % homology to said *Enterobacter aerogenes* 11E12 bacterial strain.

6. The pharmaceutical composition of claim 1 or 2, further comprising an isolated lytic bacteriophage capable of lytic infection of *Enterobacter aerogenes* 11E12 bacterial strain or a bacterial strain having greater than 85 % homology to said *Enterobacter aerogenes* 11E12 bacterial strain.

7. The pharmaceutical composition of claims 1 or 2, wherein the composition is free of lytic bacteriophage that lyse any of Klebsiella pneumoniae strain ATCC BAA-2552 (KP1), *Enterobacter aerogenes* 11E12 (KP3), ATCC 13882 (KP5), ATCC BAA-1705 (KP6), ATCC 700603 (KP7), and ATCC 700721(KP8).

8. The composition of claim 1, wherein said isolated bacteriophage has a genome which is at least 98% homologous to one of the sequences as set forth in SEQ ID NO: 21-52, as determined by BLAST.

9. The composition of claim 3, wherein said isolated bacteriophage has a genome which is at least 99 % homologous one of the sequences as set forth in SEQ ID NO: 1-19, as determined by BLAST.

10. The composition of any one of claims 1-9, for use in treating an inflammatory bowel disease.

11. The composition of claim 10, wherein the inflammatory bowel disease is ulcerative colitis.

12. The composition of claim 10, wherein the inflammatory bowel disease is Crohn's disease.

13. The composition of any one of claims 1-9, for use in treating an infection by a bacterium being a *Klebsiella pneumoniae* 2H7 bacterial strain or a bacterial strain having greater than 85 % homology to said *Klebsiella pneumoniae* 2H7 bacterial strain or a *Enterobacter aerogenes* 11E12 bacterial strain or a bacterial strain having greater than 85 % homology to said *Enterobacter aerogenes* 11E12 bacterial strain.

14. A method of determining a subject to be treated with a bacteriophage which infects a *Klebsiella pneumoniae* 2H7 bacterial strain or a bacterial strain having greater than 85 % homology to *Klebsiella pneumoniae* 2H7 bacterial strain, comprising the steps of:
a) obtaining a biological sample from the subject;
b) culturing bacteria obtained from the biological sample;
c) inoculating the cultured bacteria with at least one bacteriophage having a genomic sequence at least 90 % homologous to one of the sequences as set forth in SEQ ID NO: 21-52 and is capable of lytic infection of a *Klebsiella pneumoniae* 2H7 bacterial strain or a bacterial strain having greater than 85 % homology to said *Klebsiella pneumoniae* 2H7 bacterial strain; and
d) determining whether the cultured bacteria are lysed by the bacteriophage,
wherein when any of the cultured bacteria are lysed by the bacteriophage, the subject is determined to be infected by a *Klebsiella pneumoniae* 2H7 bacterial strain or a bacterial strain having greater than 85 % homology to said *Klebsiella pneumoniae* 2H7 bacterial strain.

15. A method of determining a subject to be treated with a bacteriophage which infects *Enterobacter aerogenes* 11E12 or a bacterial strain having greater than 85 % homology to said *Enterobacter aerogenes* 11E12 bacterial strain, comprising the steps of:
a) obtaining a biological sample from the subject;
b) culturing bacteria obtained from the biological sample;
c) inoculating the cultured bacteria with at least one bacteriophage having a genomic sequence at least 98 % homologous to one of the sequences as set forth in SEQ ID NO: 1-19 and is capable of lytic infection of *Enterobacter aerogenes* 11E12 or a bacterial strain having greater than 85 % homology to said *Enterobacter aerogenes* 11E12 bacterial strain; and
d) determining whether the cultured bacteria are lysed by the bacteriophage,
wherein when any of the cultured bacteria are lysed by the bacteriophage, the subject is determined to be infected by *Enterobacter aerogenes* 11E12 or a bacterial strain having greater than 85 % homology to said *Enterobacter aerogenes* 11E12 bacterial strain.
